# EUROPEAN PATENT SPECIFICATION

(11) **EP 4 192 839 B1**
(45) Date of publication and mention of the grant of the patent: **01.10.2025**
(21) Application number: 21756058.0
(22) Date of filing: 06.08.2021
(51) Int. Cl.: C07H 1/00, C07D 487/04, C07D 519/00, C07H 15/18, A61P 31/12, A61P 31/14

(54) **REMDESIVIR INTERMEDIATES**
REMDESIVIR-ZWISCHENPRODUKTE
INTERMÉDIAIRES DE REMDÉSIVIR

(30) Priority: 06.08.2020 HU 2000258; 10.11.2020 HU 2000371
(43) Date of publication of application: 14.06.2023
(73) Proprietor: Richter Gedeon Nyrt., 1103 Budapest (HU)
(72) Inventor: BORZA, István, 1186 Budapest (HU); UJVÁRI, Viktor, 2230 Gyömrö (HU); BANA, Péter, 5200 Törökszentmiklós (HU); PETRO, József Levente, 2440 Százhalombatta (HU); ÉLES, János, 1121 Budapest (HU); BÓDI, József, 1202 Budapest (HU); VARRÓ, Gábor, 2351 Alsónémedi (HU); KLADNI, László János, 1032 Budapest (HU); SPRÁNITZ, Péter, 1106 Budapest (HU); KRÁMOS, Balázs, 1214 Budapest (HU)
(74) Representative: Kutzenberger Wolff & Partner
(86) International application number: PCT/IB2021/057242
(87) International publication number: WO 2022/029704

(56) References cited:
- WO-A1-2009/132135
- WO-A1-2012/012776
- WO-A1-2016/069825
- DUSTIN SIEGEL ET AL: "Discovery and Synthesis of a Phosphoramidate Prodrug of a Pyrrolo[2,1- f ][triazin-4-amino] Adenine C -Nucleoside (GS-5734) for the Treatment of Ebola and Emerging Viruses", JOURNAL OF MEDICINAL CHEMISTRY, vol. 60, no. 5, 14 February 2017 (2017-02-14), US, pages 1648 - 1661, XP055489271, ISSN: 0022-2623, DOI: 10.1021/acs.jmedchem.6b01594
- HUA ZONG ET AL: "Added-Metal-Free Catalytic Nucleophilic Addition of Grignard Reagents to Ketones", THE JOURNAL OF ORGANIC CHEMISTRY, vol. 77, no. 10, 4 May 2012 (2012-05-04), pages 4645 - 4652, XP055519066, ISSN: 0022-3263, DOI: 10.1021/jo3004277

## Description

### THE FIELD OF THE INVENTION

The present invention relates to a process for the preparation of a compound of formula (I) and to novel intermediates of the synthesis.

### THE BACKGROUND OF THE INVENTION

Remdesivir (GS-5734) is a phosphoramidate prodrug of a 1'-cyano nucleoside analogue (GS-441524). The phosphoramidate prodrug approach is applied in order to mask the nucleoside to enable intracellular delivery and release of the free phosphate form (McGuigan et al., J. Med. Chem. 1993, 36(8):1048-1052). The triphosphate nucleotide form (GS-443902) resembles ATP and is used as a substrate of several viral RNA-dependent RNA polymerase (RdRp) enzymes or complexes (Cho et al., Bioorg Med Chem Lett., 2012, 22(8):2705-2707). Remdesivir is a direct-acting antiviral that inhibits RNA-dependent RNA polymerase (Gordon et al., J Biol Chem. 2020 May 15; 295(20):6785-6797) that is shown to inhibit severe acute respiratory syndrome coronavirus 2 (SARS-CoV-2), a positive-sense RNA virus, the causative agent of coronavirus disease 2019 (Covid-19), and effective in reducing the recovery time from 15 to 11 days in people hospitalized with Covid-19 (Biegel et al., NEJM, May 22, 2020; doi:10.1056/NEJMoa2007764). Remdesivir was approved for medical use in the European Union in July 2020 (see SmPC of Veklury).

The final step in the synthesis of remdesivir, i.e. the coupling reaction between the 1'-cyano nucleoside (or derivatives thereof) and the phosphoramidate prodrug moiety is well-known in the art (see e.g. CN 111471070 A; CN 111440176 A; CN 111393478 A; CN 111269263 A; CN 111233931 A; CN 111233930 A; CN 111171078 A; CN 111116656 A; WO 2016/069825 A1; WO 2012/012776 A1).

WO 2009/132135 A1 discloses the 1'-cyano nucleoside and its preparation method, wherein the coupling reaction between the protected ribonolactone and 7-bromo-pyrrolo[2,1 -*f*][1,2,4]triazin-4-ylamine is carried out by lithiation and then the cyano group is introduced as depicted in **Reaction Scheme 1:**

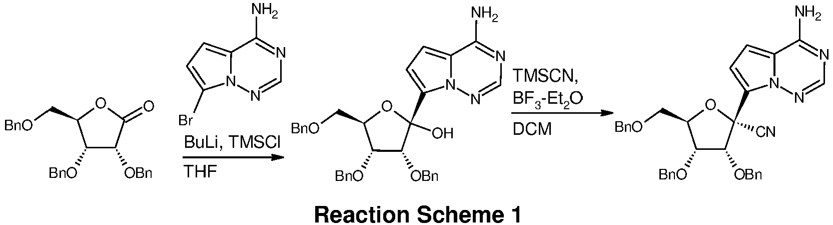

WO 2012/012776 A1 discloses the diastereomeric mixture of remdesivir and its preparation, wherein the preparation of the 1'-hydroxy nucleoside consists of the same steps as above, but trimethylsilyl trifluoromethanesulfonate (TMSOTf) was used in the cyanation step **(Reaction Scheme 2):**

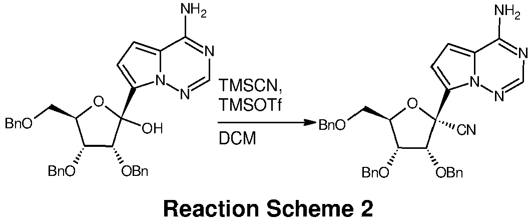

WO 2016/069825 A1 discloses the (S)-diastereomer, i.e. remdesivir and its synthetic preparation procedure. Alternative preparation methods (see 3. a)-e)) are disclosed as shown in **Reaction Scheme 3** for the formation of the 1'-hydroxy nucleoside applying *inter alia* lanthanide elements in Grignard-reaction between the protected ribonolactone and 7-iodo-pyrrolo[2,1-*f*][1,2,4]triazin-4-ylamine wherein the coupling/deprotonating agent is lithium-, or magnesium agent, such n-BuLi, i-PrMgCl, or PhMgCl, a halo-silane compound, such as TMSCI or Cl-Si(CH₃)₂(CH₂)₂Si(CH₃)₂-Cl, in the presence of a Lewis acid, such as La-, Nd, Y- or Ce-halogenides and each hydroxy group of the ribonolactone may be protected independently with a hydroxy protecting group, such as trimethylsilyl, t-butyldimethylsilyl, *t-*butyldiphenylsilyl, methyl-methoxy, tetrahydropyran, *t*-butyl, allyl, benzyl, acetyl, pivaloyl, or benzoyl, or two protecting groups on adjacent carbon atoms can be combined to form an acetonide group. The cyanation is carried out in identical circumstances as mentioned above in batch mode or by flow chemistry.

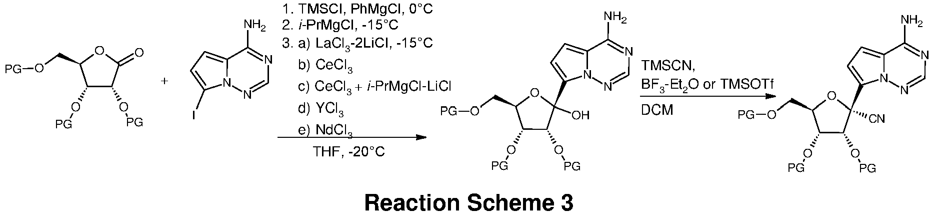

CN 111233870 A relates to a method for the preparation of pyrrolo[2,1-*f*][1,2,4]triazin-4-ylamine and its 7-substituted derivatives wherein X is halogen in high purity and yield without the need for complicated purification or separation steps as shown in **Reaction Scheme 4:**

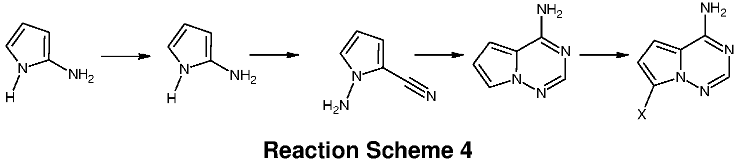

CN 111393392 A discloses a process for the preparation of the 2,3,5-tribenzyl protected ribonolactone as shown in **Reaction Scheme 5.**

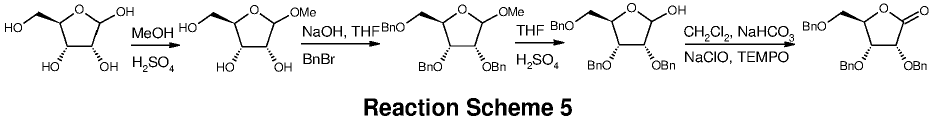

Siegel et al., J Med Chem. 2017;60(5):1648-1661 describes the synthesis optimization of remdesivir. According to the first generation synthesis depicted in **Reaction Scheme 6,** (corresponds to those described in WO 2009/132135 A1) the glycosylation reaction via metal-halogen exchange of the bromo-base followed by addition into the ribonolactone, wherein conditions a) and b) were identified to render this desired C-C bond formation, that afforded the protected ribonolactone in 25% and 60% yield, respectively. The formed 4-mono-TMS protected amino intermediate has not been isolated. The efficiency of both conditions was suboptimal as the yields were capricious and highly dependent on the cryogenic temperatures and the rate of n-BuLi addition required for the transformation. 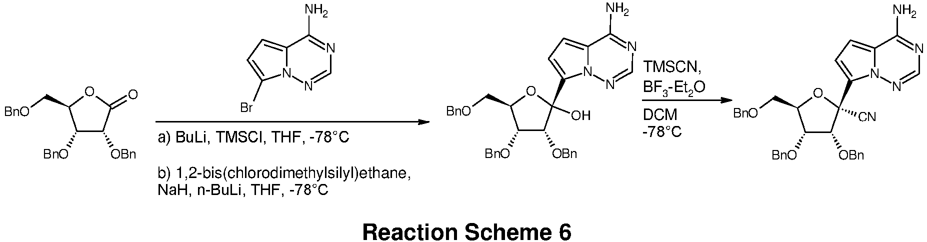 In the second generation synthesis - that enables the diastereoselective synthesis on scales suitable to advance remdesivir into preclinical studies - as shown in **Reaction Scheme** 7, the glycosylation step employed iodo-base via metal-halogen exchange with *i*-PrMgCl·LiCl complex afforded the benzyl-protected hydroxy nucleoside in a 40% yield while the subsequent cyanation afforded the benzyl-protected 1'-cyano nucleoside in 85% yield. The formed 4-mono-TMS protected amino intermediate has not been isolated. Warren et al., Nature. 2016;531(7594):381-385 (5) concerns the same preparation method also (Scheme S1-S3).

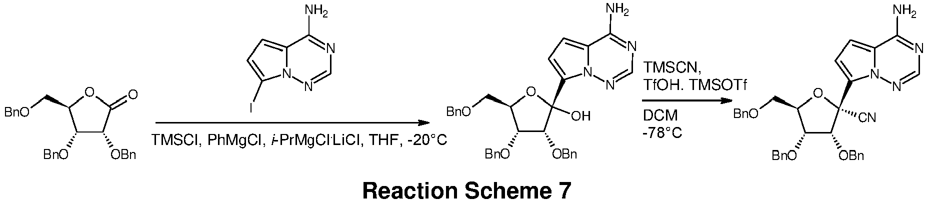

Vieira et al., OPRD, May 21, 2020 (https://dx.doi.org/10.1021/acs.oprd.0c00172) concerns a robust process for the synthesis of the key intermediates of remdesivir. Besides the batch and continuous flow chemistry processes to generate the benzyl-protected 1'-cyano nucleoside, an industrial-scale procedure for the preparation of the 1'-hydroxy nucleoside is provided (corresponding to those described in WO 2016/069825 A1) that is afforded in 69% yield when equimolar quantity of the highly expensive anhydrous neodymium-(III) chloride is used as shown in **Reaction Scheme 8.**

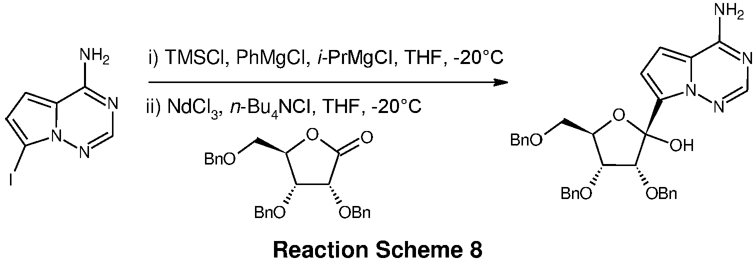

CN 111233869 A relates to alternative intermediates useful in synthesizing remdesivir. As in the glycosylation steps of **Reaction Scheme 1, 3, 6-8** the silane group protecting the 4-amino group is unstable, a novel approach is developed according to **Reaction Scheme 9** (wherein X is fluorine, chlorine, bromine or methylthio; Y is bromine or iodine; and PG is independently a hydroxyl protecting group, or two PG groups on adjacent carbon atoms together form e.g. an alkylene and the glycosylation step is carried out in the presence of magnesium or an alkylmagnesium halide, such as *i*PrMgCl, *i*PrMgCl-LiCl or PhMgCl) to circumvent the necessity of amine-protection and the difficulty of controlling the low-temperature reactions. The glycosylation and cyanation steps afforded the corresponding intermediates in good yields, but the synthesis route requires a further step introducing the 4-amine group and purification step.

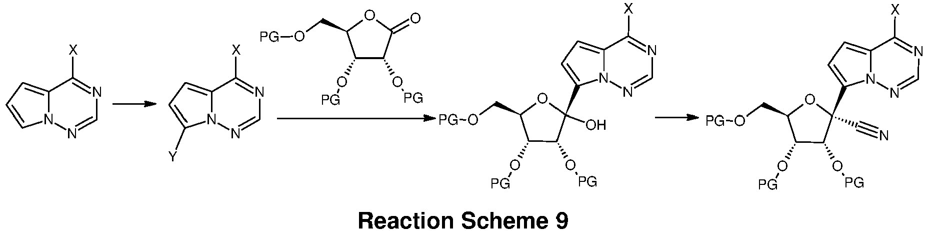

CN 111205327 A discloses a nine-step synthesis route of remdesivir, wherein - as depicted in **Reaction Scheme 10 -** in the first step the mono-BOC protected 4-amino group is formed by using a base, such as TEA, an amino protecting reagent such as (Boc)₂O, the solvent is e.g. dichloromethane and the reaction temperature is between 40 to 80°C, then in the second step one of n-BuLi, i-BuLi, LDA, iPrMgCl or iPrMgBr is used. Following the cyanation step using an alkyl cyanosilane, preferably TMSCN or TBDMSCN and removal of the 4'-benyl protecting group, the resolution of the β-anomer requires further reagents and purification steps.

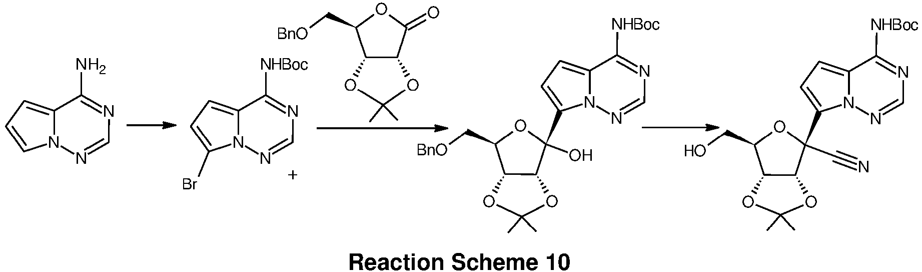

CN 111205294 A discloses yet another alternative synthesis route and intermediates thereof as shown in **Reaction Scheme 11,** wherein in the first step the benzyl-protected ribonolactone is cyanated at the 1'-position preferably in DCM, THF, or 1,2-dichloroethane at 0° to 50°C using a Lewis acid e.g. AlCl₃ and a cyano reagent such as TMSCN or TBDMSCN wherein the chiral purity is improved by applying recrystallization, to afford the compound in a 64.2%-74% yield, then glycosylation is carried out in THF or ACN using MsCl or TsCl at -10°C to 20°C in the presence of a base, e.g. TEA or DIPEA (yield 68-55%).

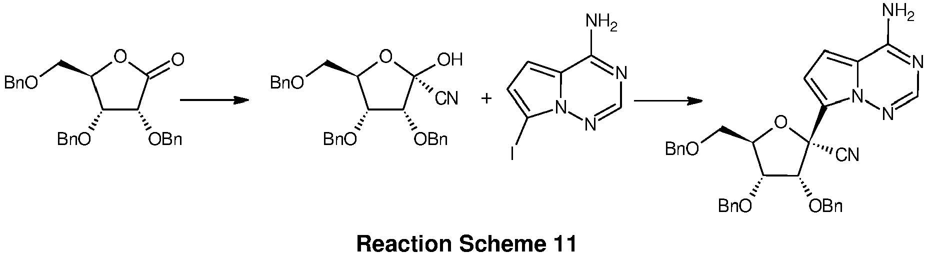

CN 111187269 A relates to a preparation method resulting in the 1'-hydroxy nucleoside by avoiding the use of the 7-iodo starting material according to **Reaction Scheme 12,** wherein in the first step the 4-amino group is protected with benzyl, Boc, Cbz, or Fmoc in DCM, THF, MeOH and/or water at 0°C to 50°C (yield: 90-86.6%); then the glycosylation is carried out in DCM/THF at -78°C to -40°C that affords the mono-protected intermediate in 47-48% yield, that requires a further deprotection step (yield: 75-82%).

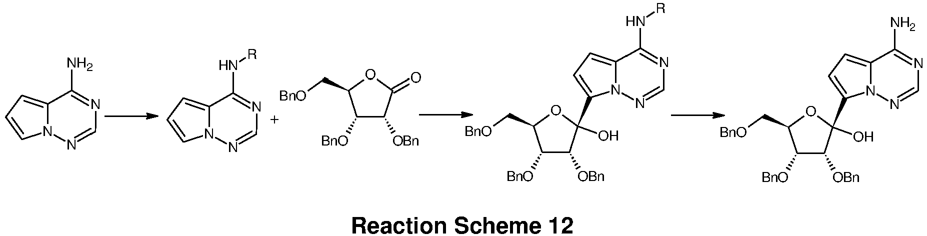

CN 110776512 A discloses a non-diastereoselective synthetic route as shown in **Reaction Scheme 13** wherein the glycosylation reaction is carried out in the manner as shown in **Reaction Scheme 1** (yield: 29.2%), the cyanation step is as shown in **Reaction Scheme 2** (yield: 85.3%) resulting in a racemic mixture which is further reacted with BCl₃ to give the 1'-cyano nucleoside (yield: 55.6%).

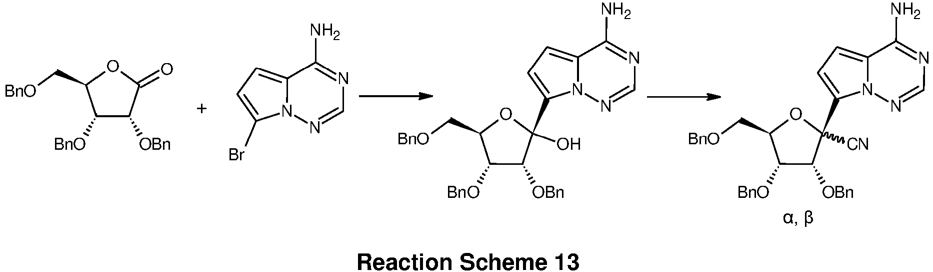

CN 110330540 A concerns a multistep synthetic route as shown in **Reaction Scheme 14** to provide the salts of the 1'-cyano nucleoside with the purpose of replacing the prodrug remdesivir. The glycosylation step is carried out between the protected ribonolactone and the 7-iodo base according to **Reaction Scheme 7** (also, the 4-mono-TMS-protected amine intermediate is formed *in situ*).

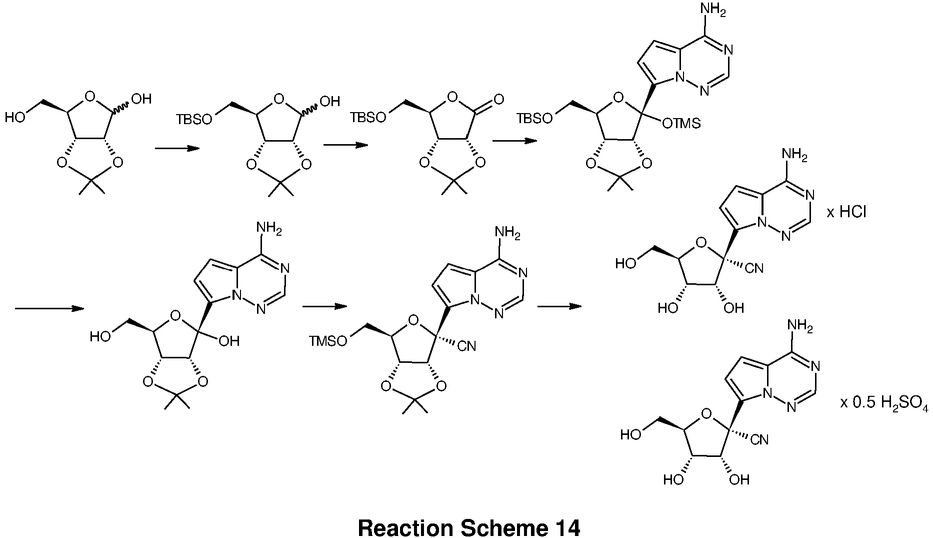

CN 111620876 A discloses a method for the preparation of the 1'-hydroxy nucleoside intermediate as shown in **Reaction Scheme** 15 wherein an organometallic compound is prepared from a halogenated base (wherein X is Cl, Br or I) by applying active zinc and iodo powder that is further reacted with the benzyl-protected ribonolactone to obtain said intermediate.

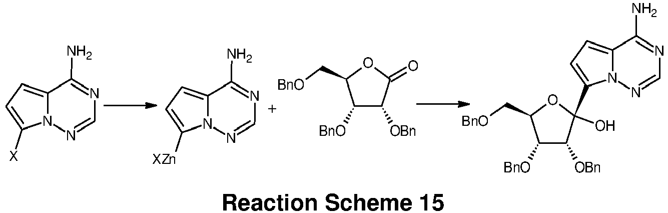

CN 111620903 A also relates to a process for the preparation of the 1'-hydroxy nucleoside wherein the coupling reaction between a base (wherein R₂, R₃ is independently H or *tert*-butoxycarbonyl group) and a protected ribonolactone (wherein Rₐ and R_{b} are both -CH₂-aryl (such as benzyl) or -Si-hydrocarbyl (such as *tert*-butyldimethyl, triisopropyl, triethyl and tert-butyldipheny) and R_{c} is alkyl or substituted phenyl) is carried out in the presence of a strong organic base containing alkali metal (such as *tert*-butyl lithium, sec-butyl lithium, lithium diisopropylamide and lithium pyrrolidine) to obtain the 1'-hydroxy nucleoside intermediate or the process (even if the 4-amino group is protected or not by means of R₂ and/or R₃) may comprise the step of adding a silane-substance to prevent any modification of the 4-amino group during the procedure, wherein said silane-substance is trimethylchlorosilane that results in a 1'-trimethylsilyl-protected analogue as shown in **Reaction Scheme 16.**

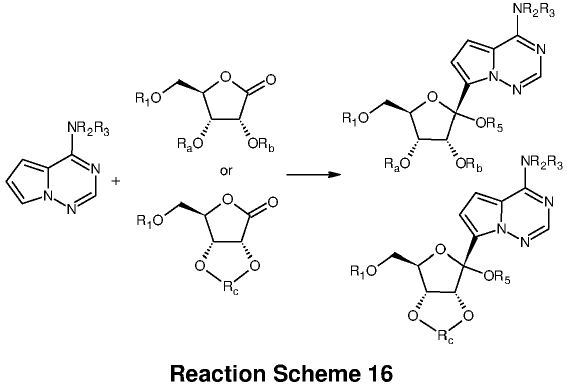

Glymes are glycole ethers, such as 1,2-dimethoxyethane (monoglyme), 1-methoxy-2-(2-methoxyethoxy)ethane (diglyme), 1-methoxy-2-[2-(2-methoxyethoxy)ethoxy]ethane (triglyme) or 1-methoxy-2-[2-[2-(2-methoxyethoxy)ethoxy]ethoxy]ethane (tetraglyme). Glymes have a broad range of industrial applications, such as their uses in cleaning products, inks, adhesives and coatings, batteries and electronics, absorption refrigeration and heat pumps, pharmaceutical formulations and also play roles in organic reactions, such as reduction, oxidation, substitution, coupling, borane chemistry, mainly as the reaction media (solvent), but can also be used as metal chelators, catalysts, reagents or reaction additives. In certain addition reactions with different Grignard reagents using NBu₄Cl as a catalyst diglyme is applied in the latter role (Huang et al,. J Org Chem 2012; 77(10):4645-4652).

As the pandemic affects millions of people wordwide, there is a need for an efficient process to produce remdesivir that allows for its production in industrial scale via intermediates with advantageous properties.

According to the procedures known in the art the provision of the 1'-cyano nucleoside in industrial scale and in higher yields is solved by various routes requiring either not easily accessible and/or expensive reagents, extreme reaction conditions and/or undesirable reaction steps but devoid of intermediates facilitating the synthesis.

Therefore, unmet need still persists to provide alternative methods for the preparation compounds of formula (I) and intermediates thereof useful in the synthesis of remdesivir and/or the 1'-cyano nucleoside thereof.

### BRIEF DESCRIPTION OF FIGURES

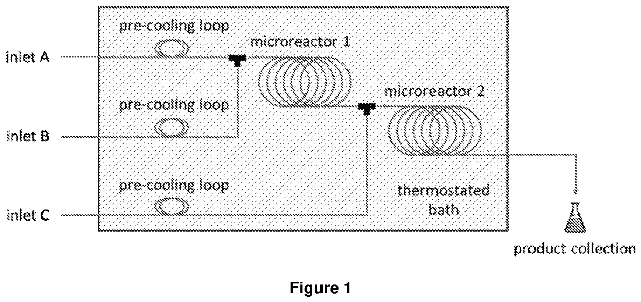

**Figure 1****:** A reagent delivery system used in the continuous flow method for the coupling reaction according to the present invention.

### SUMMARY OF THE INVENTION

The present invention relates to a process for the preparation of a compound of formula (I) comprising coupling a compound of formula (IV) with a compound of formula (III) to obtain a compound of formula (II), and cyanation of a compound of formula (II) to obtain a compound of formula (I). The process further comprises protecting a compound of formula (V) to obtain a compound of formula (IV).

The present invention also relates to a process wherein coupling a compound of formula (IV) with a compound of formula (III) in the presence of a compound of formula (VI).

The present invention also relates to the compounds of formula (II) and formula (IV).

### DETAILED DESCRIPTION OF THE INVENTION

The compounds of formula (I) known in the art are key intermediates in the synthesis of the 1'-cyano nucleoside and/or remdesivir.

The corresponding prior art procedures despite the acceptable yields have drawbacks such as the use of not easily accessible and/or expensive reagents, like (anhydrous) lantanides, extreme reaction conditions and/or undesirable reaction steps, such as the unnecessary protecting and deprotecting steps or the resolution of certain intermediates. These conditions question the industrial scale feasibility of these processes.

The present invention provides a process for the preparation of a compound of formula (I) via intermediates facilitating the synthesis, such as the compounds of formula (II) and (IV). The advantage is that such not easily accessible and/or expensive reagents are not required and/or the procedure has reduced number of reaction steps. A further advantage of the present synthesis, i.e. the application of its intermediates is that the process for the preparation of the compounds of formula (I) is industrially scalable.

The present invention relates to a process for the preparation of a compound of formula (I) comprising
step a) protecting a compound of formula (V) to obtain a compound of formula (IV),
step b1) reacting a compound of formula (IV) with a compound of formula (III) to obtain a compound of formula (II), or
step b2) carrying out step b1) in the presence of a compound of formula (VI); and
step c) cyanation of a compound of formula (II) to obtain a compound of formula (I)
according to **Reaction Scheme I** wherein X is hydrogen, bromine or iodine; R¹ is a protecting group selected from the group consisting of *t*-butyldimethylsilyl, triisopropylsilyl and *t-*butyldiphenylsilyl; R² is hydrogen or a protecting group selected from the group consisting of *t*-butyldimethylsilyl, triisopropylsilyl or *t*-butyldiphenylsilyl and PG is a protecting group selected from the group consisting of trimethylsilyl, *t*-butyldimethylsilyl, *t*-butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a -C(R³)₂- group wherein R³ is H, or C₁₋₆alkylene group.

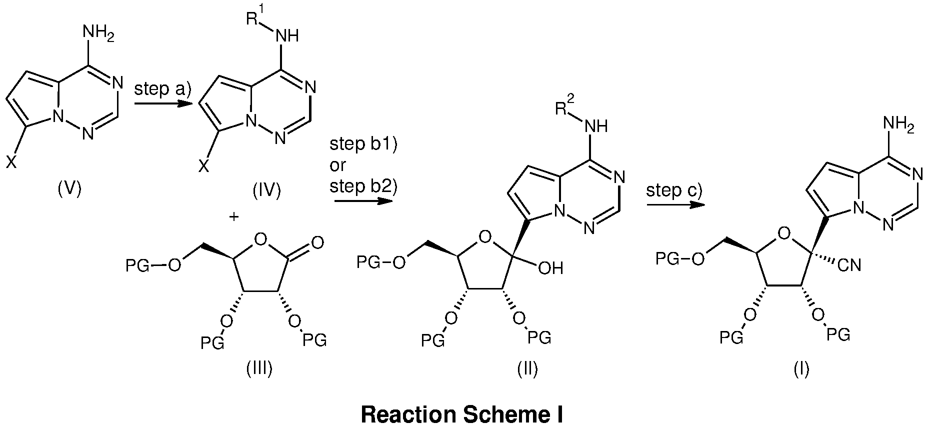

The starting materials i.e. protected ribonolactones of formula (III) or the bases of formula (V) are commercially available or can be prepared according to known procedures in the art (e.g. WO 2007/056170 A1; WO 2009/132135 A1; WO 2015/069939 A1; WO 2016/069825 A1).

In an aspect the present invention relates to a process for the preparation of a compound of formula (V) to obtain a compound of formula (IV) according to **Reaction Scheme II** wherein X is hydrogen, bromine or iodine; and R¹ is a protecting group selected from the group consisting of *t*-butyldimethylsilyl, triisopropylsilyl and *t*-butyldiphenylsilyl.

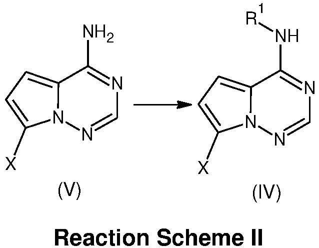

In another aspect the present invention relates to a process for the preparation of a compound of formula (II) obtained by the reaction of a compound of formula (IV) with a compound of formula (III) according to **Reaction Scheme III-a** wherein X is hydrogen, bromine or iodine; R¹ is a protecting group selected from the group consisting of *t-*butyldimethylsilyl, triisopropylsilyl and *t*-butyldiphenylsilyl; R² is hydrogen or a protecting group selected from the group consisting of *t*-butyldimethylsilyl, triisopropylsilyl and *t-*butyldiphenylsilyl and PG is a protecting group selected from the group consisting of trimethylsilyl, *t*-butyldimethylsilyl, *t*-butyldiphenylsilyl, methyl-methoxy, tetrahydropyran, *t-*butyl, allyl, benzyl, acetyl, pivaloyl and benzoyl, or two protecting groups on adjacent carbon atoms can be combined to form a -C(R³)₂- group wherein R³ is H, or C₁₋₆alkylene group.

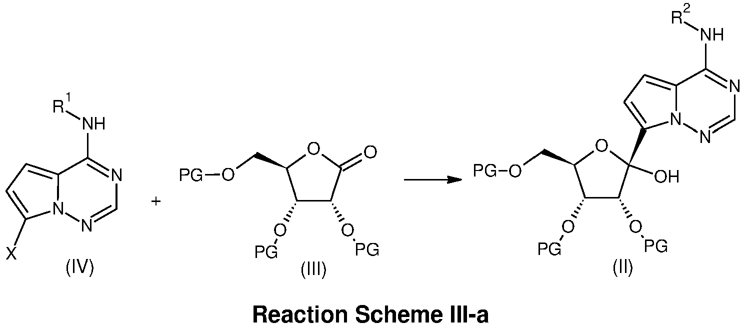

In yet another aspect the present invention relates to a process for the preparation of a compound of formula (II) obtained by the reaction of a compound of formula (IV) with a compound of formula (III) in the presence of a compound of formula (VI) according to **Reaction Scheme III-b** wherein X is hydrogen, bromine or iodine; R¹ is a protecting group selected from the group consisting of *t*-butyldimethylsilyl, triisopropylsilyl and *t-*butyldiphenylsilyl; R² is hydrogen or a protecting group selected from the group consisting of *t*-butyldimethylsilyl, triisopropylsilyl and *t*-butyldiphenylsilyl and PG is a protecting group selected from the group consisting of trimethylsilyl, *t*-butyldimethylsilyl, *t*-butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a -C(R³)₂- group wherein R³ is H, or C₁₋₆alkylene group and n is 2, 3 or 4.

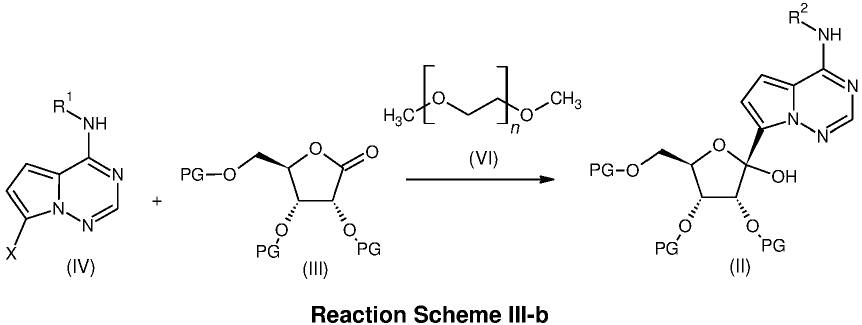

In a further aspect the present invention relates to a process for the preparation of a compound of formula (I) obtained by the cyanation of a compound of formula (II) according to **Reaction Scheme IV** wherein R² is hydrogen or a protecting group selected from the group consisting of *t*-butyldimethylsilyl, triisopropylsilyl and *t*-butyldiphenylsilyl; and PG is a protecting group selected from the group consisting of trimethylsilyl, *t*-butyldimethylsilyl, *t-*butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a - C(R³)₂- group wherein R³ is H, or C₁₋₆alkylene group.

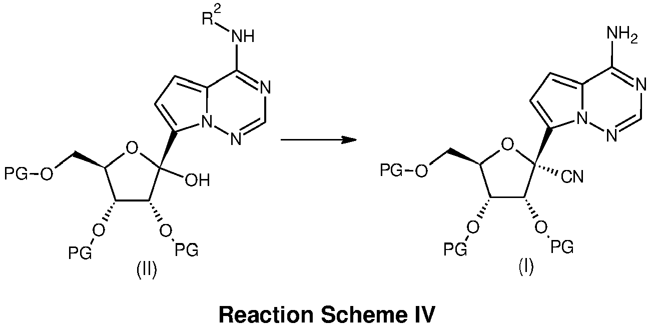

### step a) protection of 4-amino group according to Reaction Scheme II

According to step a), a compound of formula (V) is dissolved in a suitable solvent, cooled to about 0 to 5°C, then a suitable base is added, followed by the addition of a protecting agent, then work-up of the resulting reaction mixture to obtain a compound of formula (IV) wherein X is hydrogen, bromine or iodine and R¹ is a protecting group selected from the group consisting of *t*-butyldimethylsilyl, triisopropylsilyl and *t*-butyldiphenylsilyl.

The suitable solvent is a cyclic ether, such as tetrahydrofuran or 2-methyltetrahydrofuran.

The suitable base is an alkyllithium, an alkali hydroxide or an alkali alkoxide, such as n-hexyllithium, potassium-hydroxide, or potassium tert-butoxide.

The protecting agent is *t*-butyldimethylsilyl chloride, triisopropylsilyl chloride or *t-*butyldiphenylsilyl chloride.

The work-up further comprises extraction, concentration, dissolving in another suitable solvent, filtering off and washing, and/or recrystallization.

The solvent used in the recrystallization is a higher alkane, i.e. C₆₋₈alkane, such as n-hexane or n-heptane.

In a preferred embodiment the compound of formula (IV) wherein X is hydrogen and R¹ is t-butyldimethylsilyl is obtained from the compound of formula (V) wherein X is hydrogen.

Unexpectedly, it has been found that the compounds of formula (IV), compared to those reactions wheren trimethylsilyl chloride was used as protecting agent, can be isolated and remain stable that allows for the use thereof in subsequent reaction steps both in batch and continuous flow methods. Thereby, the compounds of formula (IV) allow for an efficient process to produce remdesivir and/or the 1'-cyano nucleoside in industrial scale.

A further advantage of the process is that when X is hydrogen, no halogenation, i.e. bromination or iodination thereof is required, such starting material is recoverable during the processes by which yields can be increased, furthermore, no sensitive Grignard-reaction is necessary in the next reaction step.

The present invention also relates to the compounds of formula (IV) wherein X is hydrogen, bromine or iodine; and R¹ is a protecting group selected from the group consisting of t-butyldimethylsilyl, triisopropylsilyl and *t*-butyldiphenylsilyl.

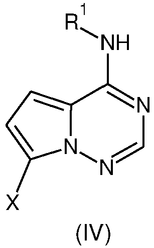

In an embodiment the present invention relates to the compounds of formula (IV) wherein X is hydrogen, bromine or iodine and R¹ is *t*-butyldimethylsilyl.

In an embodiment the present invention relates to the compounds of formula (IV) wherein X is hydrogen, bromine or iodine and R¹ is triisopropylsilyl.

In an embodiment the present invention relates to the compounds of formula (IV) wherein X is hydrogen, bromine or iodine and R¹ is *t*-butyldiphenylsilyl.

In certain embodiment the present invention relates to the compounds of formula (IV) wherein X is hydrogen or bromine and R¹ is a protecting group selected from the group consisting of *t*-butyldimethylsilyl, triisopropylsilyl and *t*-butyldiphenylsilyl.

In another embodiment the present invention relates to the compounds of formula (IV) wherein X is hydrogen or bromine and R¹ is a protecting group selected from the group consisting of triisopropylsilyl and t-butyldiphenylsilyl.

In a preferred embodiment the present invention relates to the compounds of formula (IV) selected from the group consisting of
*tert*-butyl(dimethyl)silyl-N-4-aminopyrrolo[2,1-*f*][1,2,4]triazine,
*tert*-butyl(dimethyl)silyl-N-4-amino-7-bromo-pyrrolo[2,1-*f*][1,2,4]triazine,
1,1,1-tris(propan-2-yl)-N-{pyrrolo[2,1-*f*][1,2,4]triazin-4-yl}silanamine,
N-{7-bromopyrrolo[2,1-*f*][1,2,4]triazin-4-yl}-1,1,1-tris(propan-2-yl)silanamine, and
1-*tert*-butyl-1,1-diphenyl-N-{pyrrolo[2,1-*f*][1,2,4]triazin-4-yl}silanamine.

### step b1) coupling reaction according to Reaction Scheme III-a

The reaction step b1) can be carried out in batch or continuous flow methods.

According to a batch method, a compound of formula (IV) is dissolved in a suitable solvent, to the solution a lithiation reagent is added, then a compound of formula (III) in a suitable solvent under a temperature between -78 and -50°C, followed by the work-up of the resulting reaction mixture to obtain a compound of formula (II) wherein X is hydrogen, bromine or iodine; R¹ is a protecting group selected from the group consisting of *t-*butyldimethylsilyl, triisopropylsilyl and *t*-butyldiphenylsilyl; R² is hydrogen or a protecting group selected from the group consisting of *t*-butyldimethylsilyl, triisopropylsilyl and *t-*butyldiphenylsilyl and PG is a protecting group selected from the group consisting of trimethylsilyl, *t*-butyldimethylsilyl, *t*-butyldiphenylsilyl, methyl-methoxy, tetrahydropyran, *t-*butyl, allyl, benzyl, acetyl, pivaloyl and benzoyl, or two protecting groups on adjacent carbon atoms can be combined to form a -C(R³)₂- group wherein R³ is H, or C₁₋₆alkylene group.

According to a continuous flow method, a system of two sequential continuous-flow microreactors (1 and 2) is used in an arrangement according to **Figure 1** comprising:
- microreactor 1 comprising a micromixer and a coil reactor for mixing of the N-protected substrate with the lithiation reagent and reacting them to yield the lithiated intermediate,
- microreactor 2 comprising a micromixer and a coil reactor for mixing the lithiated intermediate with the protected ribonolactone and reacting them to yield the protected product,
- input storages, pumps and injector modules providing three distinct inlets A, B and C that are connected to the two sequential microreactors,
- pre-cooling loops for each inlet, and
- a thermostated bath.

The microreactors 1 and 2 and pre-cooling loops are thermostated at -30°C.

The reaction temperature in the microreactors is -10 to -30°C.

The solution of injector A, and the solutions of injector B (microreactor 1) and injector C (microreactor 2) are sequentially injected into the flowing stream of dry THF for predetermined periods of time, followed by the collection of the mixture and an aqueous work-up thereof.

Injector A is filled with a solution of a compound of formula (IV), injector B is filled with a solution of a lithiation reagent, injector C is filled with a solution of a compound of formula (III).

The flow rate for inlet A is 1.7 mL/min, for inlet B is 0.31 mL/min and for inlet C is 0.34 mL/min.

The residence time is 20 to 45 sec for microreactor 1 and 70 to 150 sec for microreactor 2.

The suitable solvent for a compound of formula (IV) is a cyclic ether, such as tetrahydrofuran or 2-methyltetrahydrofuran.

The lithiation reagent is an alkyllithium, such as n-hexyllithium, solved in a suitable solvent, such as a higher alkane, e.g. n-hexane.

The compounds of formula (III) are protected ribonolactones wherein PG is a protecting group selected from the group consisting of trimethylsilyl, t-butyldimethylsilyl, *t-*butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a - C(R³)₂- group wherein R³ is H, or C₁₋₆alkylene group. Preferably, the compounds of formula (III) are protected ribonolactones wherein PG is a protecting group is selected from the group consisting of t-butyldimethylsilyl and benzyl, or two protecting groups adjacent carbon atoms can be combined to form a -C(R³)₂- group wherein R³ is H, or C₁₋₃alkylene group. Most preferably, the compounds of formula (III) are protected ribonolactones wherein PG is benzyl group.

The solvent of the solution of a compound of formula (III) is toluene or tetrahydrofuran.

The work-up further comprises extraction, concentration, dissolving in another suitable solvent, filtering off and washing, and/or recrystallization.

In an aspect of the invention the compounds of formula (II) are isolated.

In another aspect of the invention the compounds of formula (II) wherein R² is a protecting group selected from the group consisting of *t*-butyldimethylsilyl, triisopropylsilyl and t-butyldiphenylsilyl are reacted further without isolation thereby the coupling reaction results in the compounds of formula (II) wherein R² is hydrogen.

In an embodiment, the coupling reaction further comprises the deprotection of the amine function, using a cleaving agent, such as tetrabutylammonium fluoride trihydrate or acetic acid to obtain compounds of formula (II) wherein R² is hydrogen. Alternatively, the cleavage step is carried out without the isolation of the compounds of formula (II) wherein R² is a protecting group as mentioned above.

In an embodiment, the coupling reaction further comprises the purification of the resulting compound of formula (II) wherein R² is hydrogen.

In an embodiment, the compound of formula (II) wherein R² is hydrogen and PG is benzyl group obtained by the reaction of the compound of formula (IV) wherein X is hydrogen and R' is *t*-butyldimethylsilyl and the compound of formula (III) wherein PG is benzyl group wherein a protected compound of formula (II) is formed *in situ,* or by the deprotection without the isolation of a protected compound of formula (II).

Surprisingly, it has been found that compounds of formula (II) are obtainable in reaction circumstances between the compounds of formula (III) and (IV) that do not require either not easily accessible and/or expensive reagents, extreme reaction conditions and/or undesirable reaction steps. In addition, intermediates of formula (IV) are either isolated that is beneficial to increase yields, or suitable for carrying out the coupling, or the coupling and cyanation reactions *in situ,* thereby allowing for the reduction in the number of required reaction steps.

The present invention also relates to the compounds of formula (II) wherein R² is a protecting group selected from the group consisting of *t*-butyldimethylsilyl, triisopropylsilyl and t-butyldiphenylsilyl; and PG is a protecting group selected from the group consisting of trimethylsilyl, *t*-butyldimethylsilyl, *t*-butyldiphenylsilyl, methyl-methoxy, tetrahydropyran, *t-*butyl, allyl, benzyl, acetyl, pivaloyl and benzoyl, or two protecting groups on adjacent carbon atoms can be combined to form a -C(R³)₂- group wherein R³ is H, or C₁₋₆alkylene group.

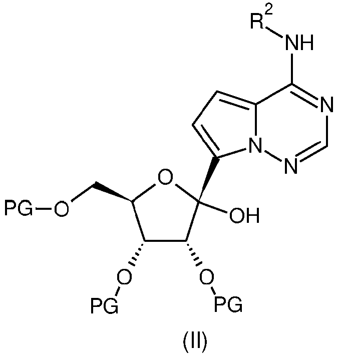

In an embodiment the present invention relates to the compounds of formula (II) wherein R² is *t*-butyldimethylsilyl and PG is a protecting group selected from the group consisting of trimethylsilyl, *t*-butyldimethylsilyl, *t*-butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a -C(R³)₂- group wherein R³ is H, or C₁₋₆alkylene group.

In an embodiment the present invention relates to the compounds of formula (II) wherein R² is triisopropylsilyl and PG is a protecting group selected from the group consisting of trimethylsilyl, *t*-butyldimethylsilyl, *t*-butyldiphenylsilyl, methyl-methoxy, tetrahydropyran, *t-*butyl, allyl, benzyl, acetyl, pivaloyl and benzoyl, or two protecting groups on adjacent carbon atoms can be combined to form a -C(R³)₂- group wherein R³ is H, or C₁₋₆alkylene group.

In an embodiment the present invention relates to the compounds of formula (II) wherein R² is *t*-butyldiphenylsilyl and PG is a protecting group selected from the group consisting of trimethylsilyl, *t*-butyldimethylsilyl, *t*-butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a -C(R³)₂- group wherein R³ is H, or C₁₋₆alkylene group.

In a preferred embodiment the present invention relates to the compounds of formula (II) wherein R² is a protecting group selected from the group consisting of *t*-butyldimethylsilyl, triisopropylsilyl and *t*-butyldiphenylsilyl; and PG is a protecting group is selected from the group consisting of *t*-butyldimethylsilyl and benzyl, or two protecting groups on adjacent carbon atoms can be combined to form a -C(R³)₂- group wherein R³ is H, or C₁₋₃alkylene group.

In another embodiment the present invention relates to the compounds of formula (II) wherein R² is a protecting group selected from the group consisting of *t*-butyldimethylsilyl, triisopropylsilyl and *t*-butyldiphenylsilyl; and PG is benzyl group.

In a preferred embodiment the present invention relates to the compounds of formula (II) selected from the group consisting of
(3*R*,4*R*,5*R*)-3,4-bis(benzyloxy)-5-[(benzyloxy)methyl]-2-{4-[(*tert*-butyldimethylsilyl)amino] pyrrolo[2,1-*f*][1,2,4]triazin-7-yl}oxolan-2-ol,
(3*R*,4*R*,5*R*)-2-{4-[(*tert*-butyldimethylsilyl)amino]pyrrolo[2,1-*f*][1,2,4]triazin-7-yl}-3,4-bis[(*tert-*butyldimethylsilyl)oxy]-5-{[(*tert*-butyldimethylsilyl)oxy]methyl}oxolan-2-ol,
(3*R*,4*R*,5*R*)-3,4-bis(benzyloxy)-5-[(benzyloxy)methyl]-2-(4-{[tris(propan-2-yl)silyl]amino} pyrrolo[2,1-*f*][1,2,4]triazin-7-yl)oxolan-2-ol,
(3a*R*,6*R*,6a*R*)-6-{[(*tert*-butyldimethylsilyl)oxy]methyl}-2,2-dimethyl-4-(4-{[tris(propan-2-yl)silyl]amino}pyrrolo[2,1-*f*][1,2,4]triazin-7-yl)-tetrahydro-2*H*-furo[3,4-d][1,3]dioxol-4-ol,
(3a*R*,6*R*,6a*R*)-6-{[(*tert*-butyldimethylsilyl)oxy]methyl}-2,2-dimethyl-4-(4-{[tris(propan-2-yl)silyl]amino}pyrrolo[2,1-*f*][1,2,4]triazin-7-yl)-tetrahydro-2*H*-furo[3,4-*d*][1,3]dioxol-4-ol,
(3*R,*4*R*,5*R*)-3,4-bis[(*tert*-butyldimethylsilyl)oxy]-5-{[(*tert*-butyldimethylsilyl)oxy]methyl}-2-(4-{[tris(propan-2-yl)silyl]amino}pyrrolo[2,1-*f*][1,2,4]triazin-7-yl)oxolan-2-ol, and
(3*R*,4*R*,5*R*)-3,4-bis(benzyloxy)-5-[(benzyloxy)methyl]-2-14-[(*tert*-butyldiphenylsilyl)amino] pyrrolo[2,1-*f*][1,2,4]triazin-7-yl}oxolan-2-ol.

### step b2) coupling reaction according to Reaction Scheme III-b

According to step b2), a compound of formula (IV) and a compound of formula (VI) wherein n is 2, 3 or 4 is dissolved in a suitable solvent, to the solution a lithiation reagent is added, then a compound of formula (III) in a suitable solvent under a temperature between -78 and -40°C, followed by the work-up of the resulting reaction mixture to obtain a compound of formula (II) wherein X is hydrogen, bromine or iodine; R¹ is a protecting group selected from the group consisting of *t*-butyldimethylsilyl, triisopropylsilyl and *t-*butyldiphenylsilyl; R² is hydrogen or a protecting group selected from the group consisting of *t*-butyldimethylsilyl, triisopropylsilyl or *t*-butyldiphenylsilyl and PG is a protecting group selected from the group consisting of trimethylsilyl, *t*-butyldimethylsilyl, *t*-butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a -C(R³)₂- group wherein R³ is H, or C₁₋₆alkylene group.

The compounds of formula (VI) are commercially available and inexpensive glycole ethers wherein n=2 denotes 1-methoxy-2-(2-methoxyethoxy)ethane (diglyme), n=3 denotes 1-methoxy-2-[2-(2-methoxyethoxy)ethoxy]ethane (triglyme) and n=4 denotes 1-methoxy-2-[2-[2-(2-methoxyethoxy)ethoxy]ethoxy]ethane (tetraglyme).

The suitable solvent for a compound of formula (IV) is a cyclic ether, such as tetrahydrofuran or 2-methyltetrahydrofuran.

The suitable solvent for a compound of formula (VI) is a cyclic ether, such as tetrahydrofuran or 2-methyltetrahydrofuran.

The lithiation reagent is an alkyllithium, such as n-hexyllithium, solved in a suitable solvent, such as a higher alkane, e.g. hexanes.

The solvent of the solution of a compound of formula (III) is a cyclic ether, such as tetrahydrofuran or 2-methyltetrahydrofuran.

The work-up further comprises extraction, concentration, dissolving in another suitable solvent, such as 2-butanone and/or a higher alkane, e.g hexanes, filtering off and washing, and/or recrystallization.

In an embodiment, the coupling reaction is carried out in the presence of a compound of formula (VI) wherein n is 2, 3 or 4. In a preferred embodiment, n is 2 or 3. In a more preferred embodiment n is 2.

In an embodiment, the coupling reaction further comprises the deprotection of the amine function, using a cleaving agent, such as tetrabutylammonium fluoride trihydrate or acetic acid to obtain compounds of formula (II) wherein R² is hydrogen. Alternatively, the cleavage step is carried out without the isolation of the compounds of formula (II) wherein R² is a protecting group as mentioned above.

In an embodiment the process further comprises crystallization wherein the reaction mixture is seeded with a corresponding compound of formula (II) wherein R² is hydrogen.

In an another embodiment the process further comprises crystallization wherein the reaction mixture is seeded with a corresponding compound of formula (II) wherein R² is a protecting group selected from the group consisting of t-butyldimethylsilyl, triisopropylsilyl and t-butyldiphenylsilyl and PG is a protecting group selected from the group consisting of trimethylsilyl, t-butyldimethylsilyl, t-butyldiphenylsilyl, methyl-methoxy, tetrahydropyran, t-butyl, allyl, benzyl, acetyl, pivaloyl and benzoyl, or two protecting groups on adjacent carbon atoms can be combined to form a -C(R³)₂- group wherein R³ is H, or C₁₋₆alkylene group.

The seed crystals of compounds of formula (II) wherein R² is hydrogen can be prepared by methods known in the art or by corresponding processes disclosed herein.

In an embodiment, the coupling reaction further comprises the purification of the resulting compound of formula (II) wherein R² is hydrogen.

In an embodiment, the compound of formula (II) wherein R² is hydrogen and PG is benzyl group obtained by the reaction of the compound of formula (IV) wherein X is hydrogen and R' is *t*-butyldimethylsilyl and the compound of formula (III) wherein PG is benzyl group wherein a protected compound of formula (II) is formed *in situ,* or by the deprotection without the isolation of a protected compound of formula (II).

In a preferred embodiment, the process for the preparation of a compound of formula (II) comprises coupling a compound of formula (IV) wherein X is hydrogen or bromine and R¹ is a protecting group selected from the group consisting of t-butyldimethylsilyl and triisopropylsilyl with a compound of formula (III) wherein PG is benzyl group in the presence of a compound of formula (VI) wherein n is 2 or 3.

In another preferred embodiment, the process for the preparation of a compound of formula (II) comprises coupling a compound of formula (IV) wherein X is hydrogen or bromine and R¹ is a protecting group selected from the group consisting of *t*-butyldimethylsilyl and triisopropylsilyl with a compound of formula (III) wherein PG is benzyl group in the presence of a compound of formula (VI) wherein n is 2 or 3 and the process further comprises crystallization wherein the reaction mixture is seeded with the corresponding compound of formula (II).

In a more preferred embodiment, the process for the preparation of a compound of formula (II) comprises coupling a compound of formula (IV) wherein X is hydrogen and R¹ is t-butyldimethylsilyl with a compound of formula (III) wherein PG is benzyl group in the presence of a compound of formula (VI) wherein n is 2.

In another more preferred embodiment, the process for the preparation of a compound of formula (II) comprises coupling a compound of formula (IV) wherein X is hydrogen and R¹ is *t*-butyldimethylsilyl with a compound of formula (III) wherein PG is benzyl group in the presence of a compound of formula (VI) wherein n is 2 and the process further comprises crystallization wherein the reaction mixture is seeded with the corresponding compound of formula (II).

Advantageously, it has been found that carrying out the coupling reaction between the compounds of formula (III) and the compounds of formula (IV) in the presence of a compound of formula (VI) unexpectedly results in increased yields of the compounds of formula (II) compared to those carried out according to step b1).

Additionally, crystallization wherein the reaction mixture is seeded with the corresponding compound of formula (II) has no or negligible effect on the yield increase as shown in Table 2.

### step c) cyanation reaction according to Reaction Scheme IV

According to step c), a compound of formula (II) wherein R² is hydrogen or a protecting group selected from the group consisting of t-butyldimethylsilyl, triisopropylsilyl or t-butyldiphenylsilyl is dissolved in a suitable solvent, such as dichloromethane, cooled under inert temperature and a Brønsted acid, such as trifluoro-acetic acid, then a solution of a Lewis acid, such as trimethylsilyl trifluoromethanesulfonate and a cyanating agent, such as trimethylsilyl cyanide in a suitable solvent, such as dichloromethane are added, followed by the work-up of the resulting reaction mixture to obtain a compound of formula (I) wherein PG is a protecting group selected from the group consisting of trimethylsilyl, *t*-butyldimethylsilyl, *t*-butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a -C(R³)₂- group wherein R³ is H, or C₁₋₆alkylene group.

The work-up further comprises extraction, concentration, dissolving in another suitable solvent, filtering off and washing, and/or recrystallization using a different solvent such as toluene.

In an aspect of the invention the cyanation reaction is carried out starting from a compound of formula (II) wherein R² is hydrogen.

In another aspect of the invention the cyanation reaction is carried out starting from a compound of formula (II) wherein R² is a protecting group selected from the group consisting of t-butyldimethylsilyl, triisopropylsilyl and t-butyldiphenylsilyl.

The term "C₁₋₆alkylene" denotes a straight or branched, single or multiple branched, hydrocarbon radical and consists of 1 to 6 carbon atoms. Examples include, but are not limited to, methylene, ethylene, propylene, i-propylene, n-butylene, 2-butylene, t-butylene, n-pentylene or n-hexylene. Preferred alkylene groups consist of 1 to 3 carbon atoms. Particularly preferred is a methylene group.

### Examples

The present invention will be further illustrated by the following Examples without limiting the scope of the present invention to them. From the above description and from the Examples, the person skilled in the art may ascertain the essential features of the invention and without departing from its essence and scope, may make certain changes and modifications in order to adapt the invention to various applications and conditions. As a result, the invention is not limited to the following illustrative examples, but rather to the scope determined by the appended claims.

**Table 1: List of abbreviations**

| **Abbreviation** | **Meaning** |
|---|---|
| AcOH | acetic acid |
| Bn | benzyl |
| DCM | dichloromethane |
| HexLi | n-Hexyllithium |
| hexanes | a mixture, composed largely (>60%) of hexane, with varying amounts of the isomeric compounds 2-methylpentane and 3-methylpentane |
| KO*t*Bu | potassium *tert*-butoxide |
| TBAF trihydrate | tetrabutylammonium fluoride trihydrate |
| TBDMS-Cl | *tert*-butyldimethylsilyl chloride |
| TBDS-Cl | *tert*-Butyldiphenylsilyl chloride |
| TFA | trifluoroacetic acid |
| THF | tetrahydrofuran |
| TIPS-Cl | triisopropylsilyl chloride |
| TMSCN | trimethylsilyl cyanide |
| TMSOTf | trimethylsilyl trifluoromethanesulfonate |

### Example 1

### Tert-butyl(dimethyl)silyl-N-4-Aminopyrrolo[2,1-f][1,2,4]triazine

4-Aminopyrrolo[2,1-*f*][1,2,4]triazine (10g, 74.54 mmol) was suspended in dry THF (80 mL) under nitrogen atmosphere. The suspension was cooled to 0-5°C and KOtBu (8.8 g, 78.27 mmol) was added and stirred for 10 minutes. After 10 minutes the solution of TBDMS-Cl (11.8g, 78.27 mmol) in dry THF (20 mL) was added dropwise. After stirring for 45 minutes the reaction was complete according to GC-MS analysis (Conversion 99%).

The reaction mixture was concentrated under reduced pressure until 55-60 g. The residue was redissolved in n-Hexane (175 mL) and it was concentrated again to 70-75 g. To the residue n-Hexane (175 mL) was added and was stirred under nitrogen atmosphere for 15 minutes. The solids were filtered off and were washed three times by 50-50 mL n-Hexane. The filtrate was concentrated under reduced pressure to 70-75 g.

The concentrated filtrate was cooled to -10°C over 40 minutes and was stirred at - 10°C for 20 minutes after the prcipitated crystals were filtered and were washed twice with 10 mL n-Hexane (-20°C). The filtrate was concentrated under reduced pressure to 5-15 g was cooled to -10°C and the crystals were filtered again.

The white crystals from both generations were dried under reduced pressure (14.11g+1.89g, 86.5%).
*¹H NMR (400 MHz, DMSO-d₆*): *δ (ppm)* 0.33 s (6H) [H₃-11, H₃-11']; 0.99 s (9H) [H₃-13, H₃-13', H₃-13"]; 6.64 dd *J* = 4.4, 2.6 Hz (1H) [H-8]; 6.89 br s (1H) [NH-10]; 7.17 dd *J* = 4.4, 1.6 Hz (1H) [H-7]; 7.64 dd *J* = 2.6, 1.6 Hz (1H) [H-9]; 7.86 s (1H) [H-3].
¹³C *NMR (100 MHz, DMSO-d₆*): *δ (ppm)* -4.1 [C-11, C-11']; 18.0 [C-12]; 26.7 [C-13, C-13', C-13"]; 102.1 [C-7]; 110.3 [C-8]; 115.9 [C-6]; 118.4 [C-9]; 147.1 [C-3]; 158.0 [C-5].
ESI-HRMS: calcd for C₁₂H₂₁N₄Si [M+H]⁺: 249.15300; found: 249.15292; delta= -0.32 ppm.

### Example 2

### Tert-butyl(dimethyl)silyl-N-4-Aminopyrrolo[2,1-f][1,2,4]triazine

4-Aminopyrrolo[2,1-*f*][1,2,4]triazine (10 g, 74.54 mmol) was suspended in dry THF (80 mL) under nitrogen atmosphere. The suspension was cooled to 0-5 °C and 2.5 M hexyllithium hexane solution was added dropwise under 15 minutes and stirred for 10 minutes. After 10 minutes the solution of TBDMS-Cl (11.8 g, 78.27 mmol) in dry THF (20 mL) was added dropwise. After stirring for 45 minutes the reaction was nearly complete according to GC-MS analysis.

The reaction mixture was concentrated under reduced pressure until 55-60 g. The residue was redissolved in n-Hexane (175 mL) and it was concentrated again to 70-75 g. To the residue n-Hexane (175 mL) was added and was stirred under nitrogen atmosphere for 15 minutes. The solids were filtered off and were washed three times by 50-50 mL n-Hexane. The filtrate was concentrated under reduced pressure to 50 g.

The concentrated filtrate was stirred under nitrogen gas and was cooled to -10°C over 40 minutes and was stirred at -10°C for 20 minutes after the precipitated crystals were filtered and were washed twice with 10 mL n-Hexane (-20°C).

The white crystals were dried under reduced pressure (12.97 g, 70.1%).

### Example 3

### Tert-butyl(dimethyl)silyl-N-4-Aminopyrrolo[2,1-f][1,2,4]triazine

4-Aminopyrrolo[2,1-*f*][1,2,4]triazine (5.00 g, 37.3 mmol) was suspended in dry THF (40 mL) under nitrogen atmosphere. The suspension was cooled to 0-5°C and 2.61 g (46.6 mmol) powdered potassium hydroxide added and stirred for 30 minutes. After 30 minutes the solution of TBDMS-Cl (7.02 g, 46.6 mmol) in dry THF (10 mL) was added dropwise. After stirring for 30 minutes the reaction was checked by TLC. Additional 1.40 g (9.29 mmol) TBDMS-Cl was added to the mixture. After 45 minutes stirring the reaction was nearly completed.

The reaction mixture was concentrated under reduced pressure until 27-30 g. The residue was suspended in n-Hexane (90 mL) and it was concentrated again to 27-30 g. To the residue n-Hexane (90 mL) was added and was stirred under nitrogen atmosphere for 15 minutes. The solids were filtered off and were washed three times by 25-25 mL n-Hexane. The filtrate was concentrated under reduced pressure to 35-37 g.

The concentrated filtrate was stirred under nitrogen gas and was cooled to -10°C over 40 minutes and was stirred at -10°C for 20 minutes after the precipitated crystals were filtered and were washed twice with 5 mL n-Hexane (-20°C). The white crystals were dried under reduced pressure.

Yield: 1.00 g (11%).

### Example 4

### Tert-butyl(dimethyl)silyl-N-4-Amino-7-bromo-pyrrolo[2,1-f][1,2,4]triazine

4-Amino-7-bromo-pyrrolo[2,1-*f*][1,2,4]triazine (7.95 g, 37.3 mmol) was suspended in 80 mL dry 2-Methyltetrahydrofuran under nitrogen atmosphere. The suspension was cooled to 0-5 °C and KOtBu (5.86 g, 52.0 mmol) was added and the suspension was stirred for 20 minutes. After 20 minutes a solution of TBDMS-Cl (7.48 g, 49.6 mmol) in dry 2-Methyltetrahydrofuran (13 mL) was added dropwise under 10 °C. After stirring for 20 minutes the reaction was nearly complete according to TLC analysis.

The reaction mixture was filtered through a celite pad. The celite pad was washed with 2-Methyltetrahydrofuran. The filtrate was concentrated under reduced pressure. The residual was suspended in 160 mL toluene. The solid substance was removed by filtration. The filtrate was evaporated under reduced pressure. The residual was stirred with n-heptane at 0-5 °C. The title product was filtered, washed with cold n-heptane. The white crystals were dried under reduced pressure.

Yield: 8.97 g (73.5%) white crystals.
*¹H NMR (500 MHz, DMSO-d₆*): *δ (ppm)* 0.33 s (6H) [H₃-13, H₃-13']; 0.98 s (9H) [H₃-12, H-12', H-12"]; 6.83 d *J* = 4.6 Hz (1H) [H-8]; 7.06 br s (1H) [NH-10]; 7.33 d *J* = 4.6 Hz (1H) [H-7]; 8.01 s (1H) [H-3].
partial ¹³C *NMR (125 MHz, DMSO-d₆*): *δ (ppm)* -4.2 [C-13, C-13']; 26.6 [C-12, C-12', C-12"]; 103.3 [C-7]; 112.5 [C-8]; 147.8 [C-3].
*ESI-LRMS* m/z(rel int%): 327(99) = [M+H]⁺; 349(3) = [M+Na]⁺. *ESI-LRMS-MS* m/z(rel int%): 311(100); 269(19).

### Example 5

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Under nitrogen a mixture of 3.00 g (12.1 mmol) *tert*-butyl(dimethyl)silyl-N-4-Aminopyrrolo[2,1-*f*][1,2,4]triazine in 30 mL dry 2-Methyltetrahydrofuran was cooled in dry ice/ethanol bath. 10.5 mL 2.3 M n-Hexyllithium solution (24.15 mmol) was added dropwise at -70 - -60 °C to the solution. After addition the mixture was stirred at -70 °C for 0.5 h. To this mixture, a solution of 5.05 g (12.1 mmol) (3R,4R,5R)-3,4-bis(benzyloxy)-5-[(benzyloxy)methyl]oxolan-2-one in 10.5 mL dry toluene was added dropwise at -70 °C. After addition the mixture was stirred at -70 °C for 0.5 h. The reaction mixture was quenched by the addition of 30 mL distilled water. The layers were separated, and 1.15 g (3.64 mmol) tetrabutylammonium fluoride trihydrate was added to the organic phase. After 20 minutes, the organic phase was washed 4 times with 30 mL 5% solution of citric acid, 30 mL saturated NaHCO₃ solution and concentrated *in vacuo* to yield 8.01 g brown oil. The crude product was dissolved in 60 mL toluene, and 28 g silica gel (Silica gel 60 (0.40-0.063 mm) Merck 1.0938530) was added. The suspension was filtered, and the silica gel was washed with additional 15-20 mL toluene. The organic phases were discarded. The silica gel was washed with 3x120 mL acetone. The combined filtrates were concentrated *in vacuo* to yield a brown oil. The product was dissolved in 30 mL methyl tert-butyl ether at 45 °C, cooled to -20 °C, and filtered to yield 3.03 g (46%) of the title compound. LC-MS (ESI) m/z 535.3 [MH-H₂O]⁺

### Example 6

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Every physical and chemical step was carried under dry nitrogen flow. To a 50 L cryogenic reactor was filled 9.0 L dry 2-Methyltetrahydrofuran, 1.62 kg (6.53 mol) *tert-*butyl(dimethyl)silyl-N-4-Aminopyrrolo[2,1-*f*][1,2,4]triazine and 7.2 L 2-Methyltetrahydrofuran. The mixture was stirred till complet dissolving. The mixture was cooled to -65 to -70 °C with liquid nitrogen.

5.4 L 33% n-Hexyllithium solution was added at -70 to -60 °C to the solution under 45-60 minutes. The pipeline was rinsed with 0.1-0.2 L hexane to the reactor. After addition the mixture was stirred at -70 °C for 0.5 h. To this mixture, a solution of 2,72 kg (6.50 mmol) (3R,4R,5R)-3,4-bis(benzyloxy)-5-[(benzyloxy)methyl]oxolan-2-one in 5.67 L dry toluene was added at -70 °C. After addition the mixture was stirred at -70 °C for 0.5 h.

To a steinless steal reactor was filled 20 L 5% citric acid solution. The cold reaction mixture was transferred into the citric acid solution. The cryogenic reactor was rinsed with 2.0 L 2-methyltetrahydrofuran into the quenched mixture. The mixture was stirred at room temperature for 10 minutes and the phases were separated. The organic phase was transferred to a glass reactor vessel.

0.82 kg (2.61 mol) TBAF trihydrate was added to the organic phase. After 30 minutes stirring at 20-25 °C, additional 0.21 kg (0.65 mol) TBAF trihydrate was added to the mixture. The endpoint of the deprotection reaction was checked by TLC. The total reaction time was 1 hour.

8.10 L 5% citric acid solution was added to the reaction mixture. The phases were settled and separeted after 10 minutes stirring. The organic phase was washed three times with 3x8.0 L 5% citric acid solution. The inorganic phases were combined and reprocessed to recover 4-aminopyrrolo[2,1-f][1,2,4]triazine.

The organic phase was washed with 4.50 L 10% sodium-hydrogencarbonate solution. After the phase separation the organic phase was evaporated in a 50 L rotary evaporator, using 45 °C water bath and (-0.8)-(-1.0) bar pressure. 6.0 L toluene was added to the residual and the evaporation was repeated. The residual was diluted to 11.3 kg with dichloromethane. The mixture was purged with chromatography.

Content of title product in the solution (measured by HPLC): 1.90-2.24 kg (53-62%)

### Example 7

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Under nitrogen to a mixture of 5.00 g (20.1 mmol) *tert*-butyl(dimethyl)silyl-N-4-Aminopyrrolo[2,1-*f*][1,2,4]triazine in 50 mL dry 2-Methyltetrahydrofuran was cooled in dry ice/ethanol bath. 17.0 mL 2.5 M n-Hexyllithium solution (42.5 mmol) was added dropwise at -70 - -65 °C. After addition the mixture was stirred at -70 °C for 0.5 h. To this mixture, a solution of 8.39 g (20.0 mmol) (3R,4R,5R)-3,4-bis(benzyloxy)-5-[(benzyloxy)methyl]oxolan-2-one in 17.5 mL dry toluene was added dropwise at -70 °C. After addition the mixture was stirred at -70 °C for 0.5 h. The reaction mixture was quenched by the addition of 2.45 mL acetic acid. The temperature was raised to 0 °C. 3.45 mL acetic acid was added to the mixture and the mixture was stirred at 55-60 °C for 1.5 h. Additional 1.00 mL acetic acid was added to the reaction mixture. The endpoint of the reaction was checked by TLC. The mixture was cooled to room temperature and it was washed 4 times with 25 mL 5% solution of citric acid, and 13 mL saturated NaHCO₃ solution and concentrated *in vacuo.* The composition of the residual corresponded with the crude product of Example 5.

### Example 8

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Every physical and chemical step was carried under dry nitrogen flow. To a 30 L jacketed glass reactor was filled 5.7 kg dry 2-Methyltetrahydrofuran, 1.20 kg (4.83 mol) *tert-*butyl(dimethyl)silyl-N-4-aminopyrrolo[2,1-*f*][1,2,4]triazine and 4.60 kg 2-Methyltetrahydrofuran. The mixture was stirred till complet dissolving. The mixture was cooled to -65 to -70 °C with -80°C jacket temperature.

2.80 L 33% n-Hexyllithium solution was added at -70 - -55 °C to the solution under 2.5-3 hours. The pipeline was rinsed with 0.25 L 2-methyltetrahydrofuran to the reactor. After addition the mixture was stirred at -60 °C to -70 °C for 0.5 h. To this mixture, a solution of 2.00 kg (4.78 mmol) (3R,4R,5R)-3,4-bis(benzyloxy)-5-[(benzyloxy)methyl]oxolan-2-one in 3.40 kg dry toluene was added under 30 minutes at -65 to -40 °C. After addition the mixture was stirred at -40 to -65 °C for 0.5 h.

0.59 L acetic acid was added to the reaction mixture at -65°C. The temperature was raised to 0 °C. Additional 0.83 L acetic acid was added to the mixture. The reaction was kept at 55 to 60 °C for 1.5 h. Additional 0.24 L acetic acid was added and the mixture was stirred at the same temperature. The endpoint of the reaction was checked by TLC.

The mixture was cooled to 20 to 23 °C. 6.00 kg 5% citric acid solution was added. After 10 minutes stirring the phases were separated. The organic phase was washed 3x6.00 kg 5% citric acid solution. The combined citric acid phases were reprocessed to recover 4-aminopyrrolo[2,1-f][1,2,4]triazine.

The organic phase was washed twice 3.70 kg 10% sodium-hydrogencarbonate solution. After the phase separation the organic phase was evaporated in a 50 L rotary evaporator, using 45 °C water bath and (-0.8)-(-1.0) bar pressure. 2.3 kg toluene was added to the residual and the evaporation was repeated. The residual was diluted to 7.70-8.00 kg with DCM. The mixture was purged with chromatography.

Content of title product in the solution (measured by HPLC): 1.44-1.45 kg (54%)

### Example 9

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

The purpose of the separation is to remove the upper impurities (protected sugar derivatives) of the crude (3*R*,4*R*,5*R*)-2-(4-aminopyrrolo[2,1-*f*][1,2,4]triazin-7-yl)-3,4-bis(benzyloxy)-5-((benzyloxy)methyl)tetrahydrofuran-2-ol (benzyl-adduct) mainly based on the TLC analysis, which can be performed at high loading with the chromatographic purification and the following crystallization.

### Chromatography conditions

The preparative chromatographic purification is conducted on a Merck Novaprep 800 type preparative HPLC instrument.
*Column:*
axial compression stainless steel column

| | |
|---|---|
| effective legth: | approx. 60 cm |
| diameter: | 5 cm |
| effective volume: | approx. 1180 cm³ |

*Operation conditions:*

| | |
|---|---|
| Stationary phase: | Merck 60 (0,040-0,063 mm) silicagel |
| mass: | 510 g |
| Eluent: | A: Dichloromethane 100 V/V %, B: Ethyl-acetate 100 V/V% |

*Gradient table*

| Time (minute) | Dichloromethane V/V% | Ethyl-acetate V/V% |
|---|---|---|
| 0 | 100 | 0 |
| 25 | 100 | 0 |
| 35 | 0 | 100 |
| 56 | 0 | 100 |

| | |
|---|---|
| Regenerating eluent: | Methanol 100 V/V% |
| Column sample loading: | 0,3 g crude benzyl-adduct / g stationary phase (165 g / 510 g) |
| Detection: | UV |
| wavelength: | 254 nm |
| sensitivity: | 3 |
| Eluent flow rate: | 100 cm³ / minute |

*Column loading*
The assembly, loading, compression of the column is conducted as the column loading description, using dichloromethane as the suspending solvent.

Materials used for column loading: 510 g silicagel and 1500 mL DCM.

### Preparation of the column prior to the separation

After loading the column is washed directly (top to bottom direction) with 1200 mL DCM. This washing step is only needed before the first injection on a freshly loaded column. For further injections the column should be regenerated and conditioned.

### Preparation of the injected sample solution

130 g crude benzyl-adduct is dissolved in 700 mL DCM. The solution is filtered on G4 glass filter, the filter is washed with 15 mL DCM, and the solutions are combined. The solution prepared this way is injected to the column continously.

### Performing the chromatography

The prepared sample solution is loaded to the prepared column through the sample feed line with a specified flow rate using the eluent pump, then additional 1800 mL DCM is pumped continously on the column through the sample feed line using the eluent pump. Thereafter a linear gradient is used, where the eluent composition is changed from 100 V/V% DCM to 100 V/V% ethyl-acetate in 10 minutes, after this 2100 mL ethyl-acetate is pumped on the column. (tₑ= elution time, tₑ=0 the starting time of the elution)

### tₑ=0- approx. 38 minutes

At approx. 38 minutes the detector signal starts to steeply ascend due to the appearence of the benzyl-adduct. The 1. fraction which contains the purified benzyl-adduct is collected from this time, approx. 38 minutes. The fraction collection of the purified benzyl-adduct lasts till approx. 56 minutes.

### te= approx. 38- approx. 56 minutes (fraction containing the purified benzyl-adduct)

The fraction collection lasts for 18 minutes, then the pump and the instrument is stopped, after this the column is regenerated. The volume of the fraction is approx. 1800 mL.

### Regeneration and conditioning of the column

The column is regenerated in countercurrent mode (bottom to top direction) with 100 mL/min flow rate using 1500 mL methanol, then the column is conditioned using 3000 mL DCM.

### Processing the clean fraction

The approx. 1800 mL volume benzyl-adduct containing fraction from the chromatographic separation is evaporated on a rotary evaporator at 40 °C and 200 mbar pressure to approx. 100-200 mL volume.

### Crystallization

The benzyl-adduct solution from the chromatography was evaporated under reduced pressure on a rotary evaporator, using 46-48 °C water bath and (-0.8)-(-1.0) bar pressure. The residual was dissolved in 650 mL methyl-tert-butyl ether at 45-50 °C. The solution was evaporated on a rotary evaporator, using 40-45 °C water bath to 520-580 mL residual. The mixture was seeded and slowly cooled down to 20-25 °C. The suspension was stirred at 20-25 °C for 2 hours and at 0-5 °C for 1 h. The product was filtered, washed with 2x40 mL cold methyl-tert-butyl ether and dried under vacuum at 40 °C.

Yield: 37-45 g white- off-white crystalls.

### Example 10

### (3R,4R,5R)-3,4-bis(benzyloxy)-5-[(benzyloxy)methyl]-2-{4-[(tert-butyldimethylsilyl)amino]pyrrolo[2,1-f][1,2,4]triazin-7-yl}oxolan-2-ol

A reagent delivery system, consisting of an Asia Pressurized Input Store, Asia Syringe Pumps and Asia Reagent Injector modules (Syrris, Royston, UK) providing three distinct inlets (A, B and C), is connected to two sequential microreactors.

Inlets A and B are connected to a PEEK T-adaptor (0.5 mm i.d; IDEX, Rohnert Park, CA, USA; Part No. P-727), followed by a coil reactor of 1 mL internal volume (PTFE tubing, 1/16 in. o.d., 0.8 mm i.d.). The outlet of the first microreactor and inlet C are connected to a second, identical PEEK T-adaptor, after which a coil reactor of 4 mL internal volume (PTFE tubing, 1/16 in. o.d., 0.8 mm i.d.) is placed. Each inlet is pre-cooled prior to the reactors in 0.5 mL volume PTFE loops (1/16 in. o.d., 0.8 mm i.d.).

The system is washed sequentially with methanol, acetone and dry THF. The reactors and pre-cooling loops are cooled to -30°C, by submerging into a thermostated IPA bath.

Injector loops are filled with the following solutions: 248 mg (1.0 mmol) of 1-*tert*-butyl-1,1-dimethyl-*N*-{pyrrolo[2,1-*f*][1,2,4]triazin-4-yl}silanamine dissolved in 5.0 mL of dry THF (0.2 M) for inlet A; 1.0 mL (2.3 mmol) of 2.3 M hexyllithium solution in hexane for inlet B; and 418 mg (1.0 mmol) of (3R,4R,5R)-3,4-bis(benzyloxy)-5-[(benzyloxy)methyl]oxolan-2-one dissolved in 1.0 mL of dry THF (1.0 M) for inlet C.

Flow rates are set to 1.7 mL/min (inlet A), 0.31 mL/min (inlet B; 2.1 eq.) and 0.34 mL/min (inlet C; 1.0 eq.), providing 0.5 min and 1.7 min residence time in the first and second microreactor. 5.0 mL of the starting material and the corresponding amounts of the other reactants are sequentially injected into the flowing stream of dry THF for predetermined periods of time. The dead volume is discarded to the waste, until the reaction mixture appears at the outlet of the second microreactor. Then, the product mixture is collected in a stirred vessel, containing distilled water.

The phases are separated, the aqueous phase is extracted with ethyl acetate. The combined organic phases are washed with brine, dried over sodium sulphate and concentrated in *vacuo* to obtain the crude product of the title compound as yellow oil. LC-MS (ESI) m/z 649.4 [M+H⁺-H₂O].

### Example 11

### (3R,4R,5R)-2-{4-[(tert-butyldimethylsilyl)amino]pyrrolo[2,1-f][1,2,4]triazin-7-yl}-3,4-bis[(tert-butyldimethylsilyl)oxy]-5-{[(tert-butyldimethylsilyl)oxy]methyl}oxolan-2-ol

Using an identical continuous-flow reactor system as in Example 10, the system is washed sequentially with methanol, acetone and dry THF. The reactors and pre-cooling loops are cooled to -30°C, by submerging into a thermostated IPA bath.

Injector loops are filled with the following solutions: 248 mg (1.0 mmol) of 1-*tert*-butyl-1,1-dimethyl-*N*-{pyrrolo[2,1-*f*][1,2,4]triazin-4-yl}silanamine dissolved in 5.0 mL of dry THF (0.2 M) for inlet A; 1.0 mL (2.3 mmol) of 2.3 M hexyllithium solution in hexane for inlet B; and 491 mg (1.0 mmol) of (3*R*,4*R*,5*R*)-3,4-bis[(*tert*-butyldimethylsilyl)oxy]-5-{[(*tert-*butyldimethylsilyl)oxy]methyl}oxolan-2-one dissolved in 1.0 mL of dry THF (1.0 M) for inlet C.

Flow rates are set to 1.7 mL/min (inlet A), 0.31 mL/min (inlet B; 2.1 eq.) and 0.34 mL/min (inlet C; 1.0 eq.), providing 0.5 min and 1.7 min residence time in the first and second microreactor. 5.0 mL of the starting material and the corresponding amounts of the other reactants are sequentially injected into the flowing stream of dry THF for predetermined periods of time. The dead volume is discarded to the waste, until the reaction mixture appears at the outlet of the second microreactor. Then, the product mixture is collected in a stirred vessel, containing distilled water.

The phases are separated, the aqueous phase is extracted with ethyl acetate. The combined organic phases are washed with brine, dried over sodium sulphate and concentrated in *vacuo* to obtain the crude product of the title compound as a yellow semisolid mixture. LC-MS (ESI) m/z 721.5 [M+H⁺-H₂O].

### Example 12

### 2-C-(4-aminopyrrolo[2,1-f][1,2,4]triazin-7-yl)-2,5-anhydro-3,4,6-tris-O-(phenylmethyl)-D-Altrononitrile

### Preparation of (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

The title product was prepared according to the procedure of Example 6.

A pilot aliquot DCM solution from the kilogram scale batch was evaporated to dryness. The residual was 13.0 g brown oil.

### Preparation of 2-C-(4-aminopyrrolo[2,1-f][1,2,4]triazin-7-yl)-2,5-anhydro-3,4,6-tris-O-(phenylmethyl)-D-Altrononitrile

Solution A: 13.0 g (23.6 mmol) crude (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol was dissolved in 130 mL DCM .The solution was cooled under nitrogen gas to -40 °C. 6.0 mL (78.3 mmol) TFA was added to the solution and it was stirred 15-20 minutes.

Solution B: In a 250 mL 3-necked flask dry DCM (65 mL) was cooled to -10°C under nitrogen atmosphere TMSOTf (26.0 mL, 143 mmol) and TMSCN (18.0 mL, 144 mmol) were added and kept on -10°C.

Solution B was added to Solution A as fast as possible while the temperature was kept under -25°C. After addition the reaction mixture was stirred on -40°C. After an hour the reaction mixture was poured on a -10 °C solution of KOH (55.0 g,) in distilled water (182 mL). The phases were separated the organic layer was washed three times with brine (3x75 mL) and twice with water (2x75 mL). The organic phase was concentrated to 83 g residual under reduced pressure. The residual was diluted with 105 mL DCM. The solution was evaporated to 80 g residual and it was diluted with 150 mL toluene. The solution was evaporated to 40 g residual. The mixture was stirred for 16 h at room temperature and 0.5 h at 0-5 °C. The product was filtered and washed with 2x2 mL 0-5 °C toluene. The crystals were dried under vacuum, nitrogen flow at room temperature.

Yield: 2.36 g white crystals.

### Example 13

### 2-C-(4-aminopyrrolo[2,1-f][1,2,4]triazin-7-yl)-2,5-anhydro-3,4,6-tris-O-(phenylmethyl)-D-Altrononitrile

### Prepearation of (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Under nitrogen a mixture of 8.50 g (26.0 mmol) *tert*-butyl(dimethyl)silyl-N-4-Amino-7-bromo-pyrrolo[2,1-*f*][1,2,4]triazine in 85 mL dry THF was cooled in dry ice/ethanol bath. 21.5 ml 2.5 M n-Hexyllithium solution (53.7 mmol) was added dropwise at -70 to -60 °C to the solution. After addition the mixture was stirred at -70 °C for 0.5 h. To this mixture, a solution of 10.90 g (26.0 mmol) (3R,4R,5R)-3,4-bis(benzyloxy)-5-[(benzyloxy)methyl]oxolan-2-one in 10.5 mL dry tetrahydrofuran was added dropwise at -70 °C. After addition the mixture was stirred at -70 °C for 0.5 h. The reaction mixture was quenched by the addition of 80 mL saturated ammonium chloride solution. The phases were separated. The organic phase was evaporated on rotary evaporator to 49 g residual. 50 mL methanol was added to the residual and the solution was evaporated to dryness under reduced pressure. Additional 45 mL methanol was added to the residual and the solution was evaporated under reduced pressure. The residual was dissolved in 100 mL methanol. 40 mL 5% citric acid solution was added to the solution and the mixture was stirred at room temperature for 0.5 h. The methanol was removed under reduced pressure. The two-phase residual was washed with 100 mL ethyl-acetate. The organic phase was diluted with 25 mL ethyl-acetate and was washed with 20 mL 5% citric acid solution and 20 mL saturated NaHCO₃ solution. The solution was evaporated to dryness under reduced pressure.

Yield: 13.4 g oil

### Preparation of 2-C-(4-aminopyrrolo[2,1-f|[1,2,4]triazin-7-yl)-2,5-anhydro-3,4,6-tris-O-(phenylmethyl)-D-Altrononitrile

This experiment was started from third part of the previously prepared crude (3*R*,4*R*,5*R*)-2-(4-aminopyrrolo[2,1-*f*][1,2,4]triazin-7-yl)-3,4-bis(benzyloxy)-5-((benzyloxy) methyltetrahydrofuran-2-ol.

Solution A: 4.47 g (8.00 mmol) crude (3R,4R,5R)-2-(4-aminopyrrolo[2,1-*f*][1,2,4]triazin-7-yl)-3,4-bis(benzyloxy)-5-((benzyloxy)methyl)tetrahydrofuran-2-ol was dissolved in 40 mL DCM .The solution was cooled under nitrogen gas to -40 °C. 1.84 mL (24.0 mmol) TFA was added to the solution and it was stirred 15-20 minutes.

Solution B: In a 100 mL 3-necked flask dry DCM (20 mL) was cooled to -10°C under nitrogen atmosphere TMSOTf (8.69 ml, 48.0 mmol) and TMSCN (6.00 ml, 48.0 mmol) were added and kept on -10°C.

Solution B was added to Solution A as fast as possible while the temperature was kept under -25°C. After addition the reaction mixture was stirred on -40°C. After an hour the reaction mixture was poured on a -10 °C solution of KOH (16.1 g,) in distilled water (65 mL). The phases were separated the organic layer was washed three times with brine (3x50 mL) and twice with water (2x20 mL). The organic phase was concentrated to dryness under reduced pressure. The residual was dissolved in toluene. The mixture was stirred for 2 days at room temperature and 0.5 h at 0-5 °C. The product was filtered and washed with 2x2 mL 0-5 °C toluene. The crystals were dried under vacuum, nitrogen flow at room temperature.

Yield: 1.57 g white crystals. (32%)

### Example 14

### N-{7-bromopyrrolo[2,1-f][1,2,4]triazin-4-yl}-1,1,1-tris(propan-2-yl)silanamine

To a stirred solution of 8 g (37.55 mmol) of 7-bromopyrrolo[2,1-*f*][1,2,4]triazin-4-amine in 5 mL of dry THF 4.49 g (40 mmol) of KOtBu was added. After solution 7.7 g (8.5 ml, 40 mmol) of TIPS-Cl was added at room temperature. The mixture was stirred at this temperature for 1.5 h. Then 0.2245 g (2 mmol) of KOtBu and 0.425 mL (2 mmol) of TIPS-Cl was added to the mixture. After stirring for 0.5 hour 0. 2245 g (2 mmol) of potassium tert-butoxide and 0.425 mL (2 mmol) of TIPS-Cl was added to the mixture again. The reaction mixture was stirred at room temperature for 1 h, diluted with water and the precipitated crystals were filtered off, washed with water. The crude product was solved in dichloromethane, washed with water, dried over anhydrous sodium sulfate, filtered and concentrated *in vacuo.* The residue was treated with n-hexane and the precipitated crystals were filtered off to yield 12.89 g (92.9 %) of the title compound.

### Example 15

### N-{7-bromopyrrolo[2,1-f][1,2,4]triazin-4-yl}-1,1,1-tris(propan-2-yl)silanamine

KOtBu (11.60 g, 103.37 mmol) was added portionwise to a suspension of compound 7-bromopyrrolo[2,1-*f*][1,2,4]triazin-4-amine (20.00 g, 93.88 mmol) in dry THF (250 mL) at 10°C. After 20 minutes TIPS-Cl (22.1 mL, 103 mmol) was added dropwise while internal temperature was kept below 10 °C. After stirring for 1 h at room temperature another amount of KOtBu (3.9 g, 34.76 mmol) and TIPS-Cl (6.6 mL, 30.84 mmol) were added at 10 °C. After stirring for 1 h at room temperature reaction was complete according to TLC analysis (eluent: cyclohexane-EtOAc 4:1). Reaction mixture was cooled to 10 °C then water (260 mL) was added (internal temperature was raised to 20-22 °C). It was extracted with EtOAc (250 mL, then 2x 100 mL). Organic layer was dried over Na₂SO₄ and concentrated under reduced pressure. Crystals were triturated with hexane, filtered and dried to give pale brown crystals (31.55 g, 91%).
*¹H NMR (500 MHz, CD₂Cl₂): δ (ppm)* 1.13 d *J* = 7.5 Hz (18H) [H₃-12, H₃-12', H₃-12", H₃-12‴, H₃-12ʺʺ, H₃-12ʺʺ′ ]; 1.49 sept *J* = 7.5 Hz (3H) [H₃-11, H-11', H-11"]; 4.74 br s (1H) [NH-10]; 6.72 m (2H) [H-7, H-8]; 7.99 s (1H) [H-3].
*¹³C NMR (125 MHz, CD₂Cl₂):δ (ppm)* 11.9 [C-11, C-11', C-11"]; 18.2 [C-12, C-12', C-12", C-12"', C-12"", C-12""']; 100.4 [C-7]; 100.7 [C-9]; 112.9 [C-8]; 117.5 [C-6]; 148.3 [C-3]; 157.8 [C-5].
*ESI-LRMS* m/z(rel int%): 369(100) = [M+H]⁺. *ESI-LRMS-MS* m/z(rel int%): 325(100).

### Example 16

### N-{7-bromopyrrolo[2,1-f][1,2,4]triazin-4-yl}-1,1,1-tris(propan-2-yl)silanamine

To a stirred suspension of 525 mg (2.46 mmol) of 7-bromopyrrolo[2,1-*f*][1,2,4]triazin-4-amine in 10 mL of dry THF 184 mg (3.28 mmol) of powdered KOH was added. A clear solution was produced from the suspension. 0.70 ml (3.28 mmol) TIPS-Cl was added at room temperature. The mixture was stirred at this temperature for 1 h. The reaction was checked by TLC. The conversion was approx. 50%. The product corresponded to the product of Example 14.

### Example 17

### 1,1,1-tris(propan-2-yl)-N-{pyrrolo[2,1-f][1,2,4]triazin-4-yl}silanamine

An oven-dried 500 mL three-necked flask equipped with an addition funnel was flushed with N₂. Starting material pyrrolo[2,1-f][1,2,4]triazin-4-amine (12.0 g; 89.5 mmol; 1.0 eq.) was added and suspended in anhydrous THF (271 ml; 0.33 M). The white suspension was cooled to 0-10°C and KOtBu (12.0 g; 107 mmol; 1.2 eq.) was added in small portions at such rate that the temperature was kept under 15°C, meanwhile the suspension turned into a greenish-yellowish solution. The mixture was stirred at 0-10°C for 15-20 min, then TIPS-Cl (22.0 ml; 103 mmol; 1.15 eq) was added dropwise via the addition funnel at such rate that the temperature was allowed to rise not higher than 25°C. After the addition was completed, conversion was monitored by TLC (cyclohexane-EtOAc 4:1; detection:UV 254nm). After 20 min, TLC showed significant starting material remained, so the reaction mixture was cooled to 0-10°C again, and another portion of KOtBu (4.0 g; 35.8 mmol; 0.4 eq.) then TIPS-Cl (6.7 ml; 31.3 mmol; 0.35 eq.) reagents were added sequentially. After stirring 20 min, TLC showed a little amount of starting material left, so the previous sequence was repeated with KOtBu (3.0 g; 26.8 mmol; 0.3 eq.) and TIPS-Cl (4.9 ml; 22.8 mmol; 0.255 eq.) After 20 min TLC showed full conversion. The solution was poured on the mixture of 200 ml cyclohexane- 200 ml water, stirred vigorously. The two layers were separated, the organic phase was washed with 100 ml brine, dried over Na₂SO₄, filtered and evaporated to dryness under reduced pressure giving 35.5 g pale yellow oil as crude product that crystallized in a few minutes. Isolation was performed by recrystallization from 48 ml n-hexane: after complete dissolution of the crude product by heating, the clear solution was cooled to -10 to 0°C and the suspension was stirred for another 1 h at this temperature. White crystals were collected by filtration, washed with 12 mL ice-cold n-hexane by obtaining 20.0 g (77%) pure product.
GC-MS (EI): *m*/*z* [M]^{*+}= 290; GC-purity: 97%
*¹H NMR (400 MHz, DMSO-d₆*): *δ (ppm)* 1.09 d *J* = 7.5 Hz (18H) [H₃-12, H₃-12', H₃-12", H₃-12‴, H₃-12ʺʺ, H₃-12ʺʺ′]; 1.43-1.54 m (3H) [H-11, H-11', H-11"]; 6.65 dd *J* = 4.4, 2.6 Hz (1H) [H-8]; 6.69 br s (1H) [NH-10]; 7.22 dd *J* = 4.4, 1.6 Hz (1H) [H-7]; 7.65 dd *J* = 2.6, 1.6 Hz (1H) [H-9]; 7.85 s (1H) [H-3].
¹³C *NMR (100 MHz, DMSO-d₆*): *δ (ppm)* 11.6 [C-11, C-11', C-11"]; 18.2 [C-12, C-12', C-12", C-12‴, C-12ʺʺ, C-12ʺʺ′]; 102.2 [C-7]; 110.2 [C-8]; 115.6 [C-6]; 118.3 [C-9]; 147.1 [C-3]; 158.2 [C-5].
*ESI-LRMS* m/z(rel int%): 291 (100) = [M+H]⁺. *ESI-LRMS-MS* m/z(rel int%): 247(100).

### Example 18

### (3R,4R,5R)-3,4-bis(benzyloxy)-5-[(benzyloxy)methyl]-2-(4-{[tris(propan-2-yl)silyl]amino}pyrrolo[2,1-f][1,2,4]triazin-7-yl)oxolan-2-ol ↔ (2R,3R,4R)-2,3,5-tris(benzyloxy)-4-hydroxy-1-(4-{[tris(propan-2-yl)silyl]amino}pyrrolo[2,1-f][1,2,4]triazin-7-yl)pentan-1-one

Under nitrogen to a solution of 500 mg (1.35 mmol) N-{7-bromopyrrolo[2,1-*f*][1,2,4]triazin-4-yl}-1,1,1-tris(propan-2-yl)silanamine in 15 mL dry THF 1.18 mL 2.3 M *n-*hexyllithium in n-hexane solution (2.71 mmol) was added dropwise at -78°C to -70°C. After addition the mixture was stirred at -78°C to -70°C for 1.5 h, 566.5 mg (1.35 mmol) (3*R*,4*R*,5*R*)-3,4-bis(benzyloxy)-5-[(benzyloxy)methyl]oxolan-2-one in THF (2 mL) solution was added dropwise at -70°C to -65°C. After addition the mixture was stirred under -70°C for 1 h. The so obtained mixture was allowed to warm to room temperature for 45 minutes. The reaction mixture was quenched by addition of 2 mL of saturated ammonium chloride solution and 30 mL of water. The reaction mixture was extracted with ethyl acetate, the combined organic layer was washed with water, dried over anhydrous sodium sulfate, filtered and concentrated *in vacuo* to obtain the crude product as an oil (63 % by LC-MS). The crude product was chromatographed on silica gel eluting with ethyl acetate and cyclohexane (1:2) to obtain the title compound as oil to yield 0.4 g (42 %). Purity by LC-MS: 97.9 %.

### Example 19

### (3R,4R,5R)-3,4-bis(benzyloxy)-5-[(benzyloxy)methyl]-2-(4-{[tris(propan-2-yl)silyl]amino}pyrrolo[2,1-f][1,2,4]triazin-7-yl)oxolan-2-ol

An oven-dried 100 mL three-necked flask under N₂-atmosphere was charged with starting material *N*-{7-bromopyrrolo[2,1-*f*][1,2,4]triazin-4-yl}-1,1,1-tris(propan-2-yl)silanamine (5.0g; 13.5 mmol; 1.0 eq) and dissolved in dry 2-Methyltetrahydrofuran (34 ml; 0.4 M). The clear solution was cooled to -70°C and n-Hexyllithium solution (11.8 ml; 2.3 M; 27.0 mmol; 2.0 eq.) was added dropwise at such rate that the temperature was kept under -55°C. After addition the mixture was stirred at -70 °C for 0.5 h. To this mixture, a solution of (3R,4R,5R)-3,4-bis(benzyloxy)-5-[(benzyloxy)methyl]oxolan-2-one (5.66 g; 13.5 mmol; 1.0 eq.) in dry toluene (12 mL; 1.0M;) was added at such rate that the temperature was kept under -55 °C. Meanwhile, the colourless solution turns into yellow. After addition the mixture was stirred at -70 °C for 0.5 h. The cold reaction mixture was poured on 50 ml 10% citric acid solution, when the organic phase turns colourless. The layers were separated, organic phase was washed with 50 mL cc. NaHCO₃ solution, 50 mL brine, dried over Na₂SO₄, filtered and evaporated to dryness under reduced pressure giving 10.75 g yellow oil. Isolation was performed by flash chromatography (100% DCM, then cyclohexane-EtOAc 4:1 -> 1:1) giving 5.0 g (52%) pale yellow oil.
LC-MS (ESI): *m*/*z* [M-H₂O+H]⁺= 691.5; purity: 98%.

### Example 20

### (3R,4R,5R)-3,4-bis(benzyloxy)-5-[(benzyloxy)methyl]-2-(4-{[tris(propan-2-yl)silyl]amino}pyrrolo[2,1-f][1,2,4]triazin-7-yl)oxolan-2-ol

Using an identical continuous-flow reactor system as in Example 10, the system is washed sequentially with methanol, acetone and dry THF. The reactors and pre-cooling loops are cooled to -30°C, by submerging into a thermostated IPA bath.

Injector loops are filled with the following solutions: 369 mg (1.0 mmol) of N-{7-bromopyrrolo[2,1-*f*][1,2,4]triazin-4-yl}-1,1,1-tris(propan-2-yl)silanamine dissolved in 5.0 mL of dry THF (0.2 M) for inlet A; 1.0 mL (2.3 mmol) of 2.3 M hexyllithium solution in hexane for inlet B; and 418 mg (1.0 mmol) of (3*R*,4*R*,5*R*)-3,4-bis(benzyloxy)-5-[(benzyloxy)methyl]oxolan-2-one dissolved in 1.0 mL of dry THF (1.0 M) for inlet C.

Flow rates are set to 1.7 mL/min (inlet A), 0.31 mL/min (inlet B; 2.1 eq.) and 0.34 mL/min (inlet C; 1.0 eq.), providing 0.5 min and 1.7 min residence time in the first and second microreactor. 5.0 mL of the starting material and the corresponding amounts of the other reactants are sequentially injected into the flowing stream of dry THF for predetermined periods of time. The dead volume is discarded to the waste, until the reaction mixture appears at the outlet of the second microreactor. Then, the product mixture is collected in a stirred vessel, containing 10% aqueous citric acid solution.

The phases are separated, the aqueous phase is extracted with ethyl acetate. The combined organic phases are washed with saturated sodium hydrogen carbonate solution, dried over sodium sulphate and evaporated. The reside is purified using flash chromatography (25% EtAc/cyclohexane) to give 133 mg of the desired product (19% yield) as a colorless oil. LC-MS (ESI) m/z 961.5 [M+H⁺-H₂O].

### Example 21

### (3R,4R,5R)-3,4-bis(benzyloxy)-5-[(benzyloxy)methyl]-2-(4-{[tris(propan-2-yl)silyl]amino}pyrrolo[2,1-f][1,2,4]triazin-7-yl)oxolan-2-ol

Using an identical continuous-flow reactor system as in Example 10, the system is washed sequentially with methanol, acetone and dry THF. The reactors and pre-cooling loops are cooled to -30°C, by submerging into a thermostated IPA bath.

Injector loops are filled with the following solutions: 290 mg (1.0 mmol) of 1,1,1-tris(propan-2-yl)-*N*-{pyrrolo[2,1-*f*][1,2,4]triazin-4-yl}silanamine dissolved in 5.0 mL of dry THF (0.2 M) for inlet A; 1.0 mL (2.3 mmol) of 2.3 M hexyllithium solution in hexane for inlet B; and 418 mg (1.0 mmol) of (3R,4R,5R)-3,4-bis(benzyloxy)-5-[(benzyloxy)methyl]oxolan-2-one dissolved in 1.0 mL of dry THF (1.0 M) for inlet C.

Flow rates are set to 1.7 mL/min (inlet A), 0.31 mL/min (inlet B; 2.1 eq.) and 0.34 mL/min (inlet C; 1.0 eq.), providing 0.5 min and 1.7 min residence time in the first and second microreactor. 5.0 mL of the starting material and the corresponding amounts of the other reactants are sequentially injected into the flowing stream of dry THF for predetermined periods of time. The dead volume is discarded to the waste, until the reaction mixture appears at the outlet of the second microreactor. Then, the product mixture is collected in a stirred vessel, containing 10% aqueous citric acid solution.

The phases are separated, the aqueous phase is extracted with ethyl acetate. The combined organic phases are washed with saturated sodium hydrogen carbonate solution, dried over sodium sulphate and evaporated. The reside is purified using flash chromatography (25% EtAc/cyclohexane) to give 99 mg of the desired product (14% yield) as a colorless oil. LC-MS (ESI) m/z 961.5 [M+H⁺-H₂O].

### Example 22

### (2R,3R,4R,5R)-3,4-bis(benzyloxy)-5-[(benzyloxy)methyl]-2-(4-{[tris(propan-2-yl)silyl]amino}pyrrolo[2,1-f][1,2,4]triazin-7-yl)oxolane-2-carbonitrile

An oven-dried 100 mL three-necked flask under N₂-atmosphere was charged with starting material (3*R*,4*R*,5*R*)-3,4-bis(benzyloxy)-5-[(benzyloxy)methyl]-2-(4-{[tris(propan-2-yl)silyl]amino}pyrrolo[2,1-*f*][1,2,4]triazin-7-yl)oxolan-2-ol (1.0 g; 1.41 mmol; 1.0 eq.) and dissolved in DCM (10 ml; 0.145M). The solution was cooled to -60 to -50°C and TFA (324 µl; 4.23 mmol; 3.0 eq.) was added dropwise During the addition white precipitate formed temporarily. Meanwhile, a separate 25 mL flask was charged with 5 mL DCM, cooled to - 10°C then TMSOTf (1.54 mL; 8.46 mmol; 6.0 eq.) and TMSCN (1.06 mL; 8.46 mmol; 6.0 eq.) were added sequentially. This TMSOTf/TMSCN solution was added to the flask containing the starting material under -50°C. During the addition the colourless solution turned yellow. The solution was stirred at -50°C for 1h, meanwhile it lightened to pale yellow. The mixture was poured on 14 mL 20% KOH-solution, the layers were separated. Inorganic phase was extracted with 1x10mL DCM, combined organic phases were washed with 1x10mL brine, dried over Na₂SO₄, filtered and evaporated to dryness under reduced pressure. Crude product was purified by flash chromatography (cyclohexane-EtOAc 3:1) by obtaining 660 mg (66%) yellow oil that crystallized in a few hours resulting in a white crystals.
LC-MS (ESI): *m*/*z* [M+H]⁺= 718.5; purity: 99%. Anomer ratio: a/b=3/1

### Example 23

### (3R,4R,5R)-2-{4-aminopyrrolo[2,1-f][1,2,4]triazin-7-yl}-3,4-bis(benzyloxy)-5-[(benzyloxy)methyl]oxolan-2-ol

Under nitrogen to a mixture of 5.00 g (17.21 mmol) 1,1,1-tris(propan-2-yl)-*N-*{pyrrolo[2,1-*f*][1,2,4]triazin-4-yl}silanamine in 50 mL dry 2-Methyltetrahydrofuran 15.5 mL 2.3 M n-Hexyllithium solution (35.6 mmol) was added dropwise at -70 °C. After addition the mixture was stirred at -70 °C for 0.5 h. To this mixture, a solution of 7.20 g (17.2 mmol) (3R,4R,5R)-3,4-bis(benzyloxy)-5-[(benzyloxy)methyl]oxolan-2-one in 15 mL dry toluene was added dropwise at -70 °C. After addition the mixture was stirred at -70 °C for 0.5 h. The reaction mixture was quenched by the addition of 40 mL distilled water. The layers were separated, and 2.17 g (6.89 mmol) TBAF trihydrate was added to the organic phase. After 0.5 h, the organic phase was washed with a solution of citric acid, saturated NaHCO₃ solution and concentrated *in vacuo* to yield 12.50 g brown oil. The crude product was dissolved in 100 mL toluene, and 43 g silica gel was added. The suspension was filtered, and the silica gel was washed with additional 100 mL toluene. The organic phases were discarded. The silica gel was washed with 2x175 mL, then 100 mL acetone. The combined filtrates were concentrated *in vacuo* to yield 6.80 g brown oil. The product was dissolved in 34 mL methyl tert-butyl ether at 45 °C, cooled to -20 °C, and filtered to yield 1.97 g (20.6%) of the title compound. LC-MS (ESI) m/z 535.3 [MH-H₂O]⁺

### Example 24

### (3R,4R,5R)-2-{4-aminopyrrolo[2,1-f][1,2,4]triazin-7-yl}-3,4-bis(benzyloxy)-5-[(benzyloxy)methyl]oxolan-2-ol

Under nitrogen to a mixture of 5.00 g (13.54 mmol) *N*-{7-bromopyrrolo[2,1-*f*][1,2,4]triazin-4-yl}-1,1,1-tris(propan-2-yl)silanamine in 50 mL dry 2-Methyltetrahydrofuran 12.2 mL 2.3 M n-Hexyllithium solution (28 mmol) was added dropwise at -70 °C. After addition the mixture was stirred at -70 °C for 0.5 h. To this mixture, a solution of 5.66 g (13.5 mmol) (3*R*,4*R*,5*R*)-3,4-bis(benzyloxy)-5-[(benzyloxy)methyl]oxolan-2-one in 10 mL dry toluene was added dropwise at -70 °C. After addition the mixture was stirred at -70 °C for 0.5 h. The reaction mixture was quenched by the addition of 40 mL distilled water. The layers were separated, and 1.71 g (5.41 mmol) TBAF trihydrate was added to the organic phase. After 0.5 h, the organic phase was washed with a solution of citric acid, saturated NaHCO₃ solution and concentrated *in vacuo* to yield 10.49 g brown oil. The crude product was dissolved in 100 mL toluene, and 37 g silica gel was added. The suspension was filtered, and the silica gel was washed with additional 100 mL toluene. The organic phases were discarded. The silica gel was washed with 2x175 mL, then 100 mL acetone. The combined filtrates were concentrated *in vacuo* to yield 6.57 g brown oil. The product was dissolved in 33 mL methyl tert-butyl ether at 45 °C, cooled to -20 °C, and filtered to yield 2.81 g (37.5%) of the title compound. LC-MS (ESI) m/z 535.3 [MH-H₂O]⁺

### Example 25

### (3aR,6R,6aR)-6-{[(tert-butyldimethylsilyl)oxy]methyl}-2,2-dimethyl-4-(4-{[tris(propan-2-yl)silyl]amino}pyrrolo[2,1-f][1,2,4]triazin-7-yl)-tetrahydro-2H-furo[3,4-d][1,3]dioxol-4-ol

Under nitrogen to a solution of 2 g (5.41 mmol) N-{7-bromopyrrolo[2,1-*f*][1,2,4]triazin-4-yl}-1,1,1-tris(propan-2-yl)silanamine in 30 mL dry THF 4.71 mL 2.3 M n-hexyllithium in *n-*hexane solution (4.71 mmol) was added dropwise at (-78) °C - (-70) °C. After addition the mixture was stirred at -78 °C to -70 °C for 0.5 h, 1.64 g (5.41 mmol) (3a*R,*6*R*,6*a*R)-6-{[(*tert-*butyldimethylsilyl)oxy]methyl}-2,2-dimethyl-tetrahydro-2*H*-furo[3,4-*d*][1,3]dioxol-4-one in THF (5 mL) solution was added dropwise at -70 °C to -65 °C. After addition the mixture was stirred under -70°C for 1 h. The so obtained mixture was allowed to warm to room temperature for 45 minutes. The reaction mixture was quenched by addition of 5 mL of saturated ammonium chloride solution and 30 mL of water. The reaction mixture was extracted with ethyl acetate, the combined organic layer was washed with water, dried over anhydrous sodium sulfate, filtered and concentrated *in vacuo* to obtain 3.6 g of the crude product of the title compound as an oil (75 % by LC-MS).

### Example 26

### (3aR,6R,6aR)-6-{[(tert-butyldimethylsilyl)oxy]methyl}-2,2-dimethyl-4-(4-{[tris(propan-2-yl)silyl]amino}pyrrolo[2,1-f][1,2,4]triazin-7-yl)-tetrahydro-2H-furo[3,4-d][1,3]dioxol-4-ol

Using an identical continuous-flow reactor system as in Example 10, the system is washed sequentially with methanol, acetone and dry THF. The reactors and pre-cooling loops are cooled to -30°C, by submerging into a thermostated IPA bath.

Injector loops are filled with the following solutions: 369 mg (1.0 mmol) of *N*-{7-bromopyrrolo[2,1-*f*][1,2,4]triazin-4-yl}-1,1,1-tris(propan-2-yl)silanamine dissolved in 5.0 mL of dry THF (0.2 M) for inlet A; 1.0 mL (2.3 mmol) of 2.3 M hexyllithium solution in hexane for inlet B; and 302 mg (1.0 mmol) of (3a*R*,6*R*,6a*R*)-6-{[(*tert*-butyldimethylsilyl)oxy]methyl}-2,2-dimethyl-tetrahydro-2*H*-furo[3,4-*d*][1,3]dioxol-4-one dissolved in 1.0 mL of dry THF (1.0 M) for inlet C.

Flow rates are set to 1.7 mL/min (inlet A), 0.31 mL/min (inlet B; 2.1 eq.) and 0.34 mL/min (inlet C; 1.0 eq.), providing 0.5 min and 1.7 min residence time in the first and second microreactor. 5.0 mL of the starting material and the corresponding amounts of the other reactants are sequentially injected into the flowing stream of dry THF for predetermined periods of time. The dead volume is discarded to the waste, until the reaction mixture appears at the outlet of the second microreactor. Then, the product mixture is collected in a stirred vessel, containing 10% aqueous citric acid solution.

The phases are separated, the aqueous phase is extracted with ethyl acetate. The combined organic phases are washed with saturated sodium hydrogen carbonate solution, dried over sodium sulphate and concentrated in *vacuo* to obtain 562 mg of the crude product of the title compound as yellow oil. LC-MS (ESI) m/z 575.4 [M+H⁺].

### Example 27

### (3R,4R,5R)-3,4-bis[(tert-butyldimethylsilyl)oxy]-5-{[(tert-butyldimethylsilyl)oxy]methyl}-2-(4-{[tris(propan-2-yl)silyl]amino}pyrrolo[2,1-f][1,2,4]triazin-7-yl)oxolan-2-ol

Under nitrogen to a solution of 500 mg (1.35 mmol) N-{7-bromopyrrolo[2,1-*f*][1,2,4]triazin-4-yl}-1,1,1-tris(propan-2-yl)silanamine in 15 mL dry THF 1.18 mL 2.3 M *n-*hexyllithium in *n*-hexane solution (2.71 mmol) was added dropwise at -78 °C to -70 °C. After addition the mixture was stirred at -78 °C to -70 °C for 1.5 h, 664 mg (1.35 mmol) (3R,4R,5R)-3,4-bis[(*tert*-butyldimethylsilyl)oxy]-5-{[(*tert*-butyldimethylsilyl)oxy]methyl}oxolan-2-one in THF (2 mL) solution was added dropwise at -70 °C to -65 °C. After addition the mixture was stirred under -70 °C for 1 h. The so obtained mixture was allowed to warm to room temperature for 45 minutes. The reaction mixture was quenched by addition of 2 mL of saturated ammonium chloride solution and 30 mL of water. The reaction mixture was extracted with ethyl acetate, the combined organic layer was washed with water, dried over anhydrous sodium sulfate, filtered and concentrated *in vacuo* to obtain 0.9 g of the crude product of the title compound as brown oil (55 % by LC-MS).

### Example 28

### (3R,4R,5R)-3,4-bis[(tert-butyldimethylsilyl)oxy]-5-{[(tert-butyldimethylsilyl)oxy]methyl}-2-(4-{[tris(propan-2-yl)silyl]amino}pyrrolo[2,1-f][1,2,4]triazin-7-yl)oxolan-2-ol

Using an identical continuous-flow reactor system as in Example 1, the system is washed sequentially with methanol, acetone and dry THF. The reactors and pre-cooling loops are cooled to -30°C, by submerging into a thermostated IPA bath.

Injector loops are filled with the following solutions: 369 mg (1.0 mmol) of *N*-{7-bromopyrrolo[2,1-*f*][1,2,4]triazin-4-yl}-1,1,1-tris(propan-2-yl)silanamine dissolved in 5.0 mL of dry THF (0.2 M) for inlet A; 1.0 mL (2.3 mmol) of 2.3 M hexyllithium solution in hexane for inlet B; and 490 mg (1.0 mmol) of (3R,4R,5R)-3,4-bis[(tert-butyldimethylsilyl)oxy]-5-{[(*tert-*butyldimethylsilyl)oxy]methyl}oxolan-2-one dissolved in 1.0 mL of dry THF (1.0 M) for inlet C.

Flow rates are set to 1.7 mL/min (inlet A), 0.31 mL/min (inlet B; 2.1 eq.) and 0.34 mL/min (inlet C; 1.0 eq.), providing 0.5 min and 1.7 min residence time in the first and second microreactor. 5.0 mL of the starting material and the corresponding amounts of the other reactants are sequentially injected into the flowing stream of dry THF for predetermined periods of time. The dead volume is discarded to the waste, until the reaction mixture appears at the outlet of the second microreactor. Then, the product mixture is collected in a stirred vessel, containing 10% aqueous citric acid solution.

The phases are separated, the aqueous phase is extracted with ethyl acetate. The combined organic phases are washed with saturated sodium hydrogen carbonate solution, dried over sodium sulphate and evaporated. The reside is purified using flash chromatography (6% EtAc/cyclohexane) to give 60 mg of the desired product (8% yield) as a colorless oil. LC-MS (ESI) m/z 763.6 [M+H⁺-H₂O].

### Example 29

### 1-Tert-butyl-1,1-diphenyl-N-{pyrrolo[2,1-f][1,2,4]triazin-4-yl}silanamine

KOtBu (5.52 g, 49.20 mmol) was added portionwise to a suspension of compound pyrrolo[2,1-*f*][1,2,4]triazin-4-amine (6.00 g, 44.73 mmol) in dry THF (171 mL) at 10 °C. After 20 minutes TBDPS-Cl (12.8 mL, 49.20 mmol) was added dropwise while internal temperature was kept below 10 °C. After stirring for 40 min at rt another amount of potassium *tert*-butoxide (1.7 g, 15.15 mmol) and TBDPS-Cl (3.85 mL, 14.81 mmol) were added at 10 °C. After stirring for 40 min at rt reaction was complete according to TLC analysis (eluent: cyclohexane-EtOAc 4:1). Reaction mixture was poured into mixture of EtOAc (171 mL) and ice-cold water (171 mL). Phases were separated and aqueous phase was extracted with EtOAc (2x 100 mL). Organic layer was dried over Na₂SO₄ and concentrated under reduced pressure. To the crude product toluene (240 mL) was added and it was stirred to 10 minutes. It was filtered and filtrate was concentrated under reduced pressure. Crystals were triturated with hexane, filtered and dried to obtain white crystals (12.15 g, 73%).
¹H NMR (500 MHz, DMSO-*d*₆): δ (ppm) 1.07 s (9H) [H₃-13, H₃-13', H₃-13"]; 6.73 dd *J* = 4.4, 2.6 Hz (1H) [H-8]; 7.27 s (1H) [H-10]; 7.35-7.44 m (6H) [H-16, H-16', H-16", H-16"', H-17, H-17']; 7.45 dd *J* = 4.4, 1.5 Hz (1H) [H-7]; 7.61 s (1H) [H-3]; 7.62-7.67 m (4H) [H-15, H-15', H-15", H-15"]'; 7.71 dd *J* = 2.6, 1.5 Hz (1H) [H-9].
¹³C{¹H} NMR (126 MHz, DMSO-*d*₆): δ (ppm) 18.1 [C-12]; 27.4 [C-13, C-13', C-13"]; 102.8 [C-7]; 110.6 [C-8]; 115.6 [C-6]; 118.6 [C-9]; 127.5 [C-16, C-16', C-16", C-16"']; 129.2 [C-17, C-17']; 133.1 [C-14, C-14']; 134.7 [C-15, C-15', C-15", C-15"']; 146.5 [C-3]; 157.5 [C-5].
*ESI-LRMS* m/z(rel int%): 373(100) = [M+H]⁺; 395(2) = [M+Na]⁺. *ESI-LRMS-MS* m/z(rel int%): 295(100).

### Example 30

### (3R,4R,5R)-3,4-bis(benzyloxy)-5-[(benzyloxy)methyl]-2-{4-[(tert-butyldiphenylsilyl)amino]pyrrolo[2,1-f][1,2,4]triazin-7-yl}oxolan-2-ol

Under inert atmosphere 1-*tert*-butyl-1,1-diphenyl-*N*-{pyrrolo[2,1-*f*][1,2,4]triazin-4-yl}silanamine (600 mg, 1.61 mmol) was dissolved in dry THF (15 mL) and cooled below -60 °C. n-Hexyllithium in hexane (2.3 M, 1.4 mL) was added dropwise while internal temperature was kept below -60 °C. After stirring for 1 h at this temperature (3*R*,4*R*,5*R*)-3,4-bis(benzyloxy)-5-((benzyloxy)methyl)tetrahydrofuran-2(3*H*)-one (674 mg, 1.61 mmol) in dry THF (2 mL) was added and it was stirred for 1 h below -60 °C. Reaction mixture was allowed to warm to 0 °C and saturated aqueous NH₄Cl solution (3.5 mL) and water (10 mL) was added. It was extracted with EtOAc (4x 20 mL). Organic layer was dried over Na₂SO₄ and concentrated under reduced pressure. Protected product was purified with flash chromatography (eluent cyclohexane-EtOAc 100:0 to 80:20) to give colourless oil (998.4 mg). LC-MS (ESI) m/z 773.4 [MH-H₂O]⁺.

### Example 31

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

(3*R,*4*R*,5*R*)-3,4-bis(benzyloxy)-5-[(benzyloxy)methyl]-2-{4-[(*tert*-butyldiphenylsilyl) amino]pyrrolo[2,1-*f*][1,2,4]triazin-7-yl}oxolan-2-ol (998.4 mg, 1.26 mmol) was dissolved in 2-methyltetrahydrofuran (20 mL) and TBAF trihydrate (230 mg, 0,73 mmol) was added and stirred for 30 minutes at room temperature. Completion of deprotection was followed by TLC (eluent cyclohexane-EtOAc 4:1). Toluene (20 mL) was added and organic phase was washed with water (3x 25 mL). Organic layer was dried over Na₂SO₄ and concentrated under reduced pressure to give yellow oil (1.20 g) as crude product. It was dissolved in toluene (15 mL) and silica gel (4.2 g) was added then stirred at rt for 30 min. Silica gel was filtered out, washed with toluene. Organic phases were discarded. Then silica gel was washed with acetone (3x 50 mL) in order to obtain crude product from silica. Organic fractions were collected and concentrated under reduced pressure. It was dissolved in methyl tert-buthyl ether (4 ml) at 40 °C and seeded at rt. It was cooled to -20 °C and stirred for 2 hours. Crystals were filtered, washed with methyl *tert*-buthyl ether and dried to dryness to obtain white crystals (495.2 mg, 55% for two reaction step). LC-MS (ESI) m/z 553.6 [MH⁺].

### Example 32

### (3R,4R,5R)-3,4-bis(benzyloxy)-5-[(benzyloxy)methyl]-2-{4-[(tert-butyldiphenylsilyl)amino]pyrrolo[2,1-f][1,2,4]triazin-7-yl}oxolane-2-carbonitrile

Solution A: Under inert atmosphere TMSOTf (1.90 mL, 10.43 mmol) was dissolved in DCM (6 mL) at -10 °C. TMSCN (1.31 mL, 10.48 mmol) was added and stirred at this temperature. Solution B: Under inert atmosphere (3*R*,4*R*,5*R*)-3,4-bis(benzyloxy)-5-[(benzyloxy)methyl]-2-{4-[(*tert*-butyldiphenylsilyl)amino]pyrrolo[2,1-*f*][1,2,4]triazin-7-yl}oxolan-2-ol (1379 mg, 1.74 mmol) was dissolved in DCM (12 mL) and cooled below -50 °C. TFA (0.40 mL, 5.23 mmol) was added and it was stirred for 10 min. Then solution A was added while internal temperature was kept below -50 °C. After stirring for 1 h at this temperature it was poured into aqueous KOH solution (prepared from KOH (3.52 g) and water (14 mL)). Phases were separated and aqueous layer was washed with dichloromethane (3x 15 mL). Combined organic phase was washed with water (15 mL), dried over Na₂SO₄ and concentrated under reduced pressure. It was purified with flash chromatography (eluent: cyclohexane-EtOAc 100:0 to 90:10) to give 314.2 mg (1.74 mmol, 23%) white foam as title product as α-anomer. LC-MS (ESI) m/z 800.5 [MH⁺].

### Example 33

### 2-C-(4-aminopyrrolo[2,1-f][1,2,4]triazin-7-yl)-2,5-anhydro-3,4,6-tris-O-(phenylmethyl)-D-Altrononitrile

Solution A: (3*R*,4*R*,5*R*)-2-(4-aminopyrrolo[2,1-*f*][1,2,4]triazin-7-yl)-3,4-bis(benzyloxy)-5-((benzyloxy)methyl)tetrahydrofuran-2-ol (35 g, 63 mmol) was measured in a 1000 mL 3-necked flask under nitrogen atmosphere and was dissolved in dry DCM (350 mL) and the solution was cooled to -40°C. TFA (14.5 mL) was added to the stirred solution.

Solution B: In a 500 mL 3-necked flask dry DCM (130 mL) was cooled to -10°C under nitrogen atmosphere TMSOTf (69 mL, 380 mmol) and TMSCN (48 mL, 380 mmol) were added and kept on -10°C.

Solution B was added to solution A as fast as possible while the temperature was kept under -25°C. After addition the reaction mixture was stirred on -40°C. After an hour the reaction mixture was poured on a -10°C solution of KOH (127g, 2267 mmol) in water (490 mL). The phases were separated the organic layer was washed three times with brine (3x180 mL) and twice with water (2x180 mL). The organic phase was concentrated to 210-220 g. To the concentrated solution DCM (280 mL) was added and it was concentrated to 200-315 g where the concentrated solution was filtered and was further concentrated to 210-220 g. To the concentrated solution Toluene (665 mL) was added and it was concentrated to 440-470 g then the suspension was placed on a 90°C bath until the crystals were redissolved. At this point the heating was turned down to 55°C until crystallization started when the heating was turned off and the stirred suspension was let cool to room temperature. The crystals were filtered on room temperature they were washed with cold Toluene and were dried under reduced pressure to obtain white crystals (24.79 g, 69.7%)

### Example 34

### 2-C-(4-aminopyrrolo[2,1-f][1,2,4]triazin-7-yl)-2,5-anhydro-3,4,6-tris-O-(phenylmethyl)-D-Altrononitrile

Solution A: Dry DCM (1000 mL) was measured in a 2000 mL reaction vessel under nitrogen atmosphere and (3*R*,4*R*,5*R*)-2-(4-aminopyrrolo[2,1-*f*][1,2,4]triazin-7-yl)-3,4-bis(benzyloxy)-5-((benzyloxy)methyl)tetrahydrofuran-2-ol (100 g, 181 mmol) was dissolved the solution was cooled to -40°C. TFA (42 mL) was added to the stirred solution.

Solution B: In a 1000 mL 3-necked flask dry DCM (500 mL) was cooled to -10°C under nitrogen atmosphere TMSOTf (242 g, 1090 mmol) and TMSCN (242 mL, 1090 mmol) were added and kept on -10°C.

Solution B was added to solution A as fast as possible while the temperature was kept under -25°C. After addition the reaction mixture was stirred on -40°C. After an hour the reaction mixture was poured on a -10°C solution of KOH (182 g, 3240 mmol) in water (700 mL). The phases were separated the organic layer was washed three times with brine (3x540 mL) and twice with water (2x540 mL). The organic phase was concentrated to about 600 mL. To the concentrated solution DCM (800 mL) was added and it was concentrated to about 650 mL where the concentrated solution was filtered and was further concentrated to 450 mL. To the concentrated solution Toluene (1900 mL) was added and it was concentrated to 1200 mL then the suspension was heated to 90°C until the crystals were redissolved. The solution was cooled to 55°C in 30 minutes. The resulting suspension was cooled to 22°C over 90 minutes.

The crystals were filtered, washed with Toluene (200 mL in three portions) and was dried to dryness giving white crystals (76.48g, 74.98%).

### Example 35

### 2-C-(4-aminopyrrolo[2,1-f][1,2,4]triazin-7-yl)-2,5-anhydro-3,4,6-tris-O-(phenylmethyl)-D-Altrononitrile

Solution A: Solution of (3*R*,4*R*,5*R*)-2-(4-aminopyrrolo[2,1-*f*][1,2,4]triazin-7-yl)-3,4-bis(benzyloxy)-5-((benzyloxy)methyl)tetrahydrofuran-2-ol (3 kg) in dry DCM (40.8 kg) was cooled to -40°C and TFA (2 kg) was charged under inert atmosphere.

Solution B: To pre-cooled (-10°C) dry DCM (20.4 kg) TMSOTf (7.2 kg) and TMSCN (3.2 kg) was charged under inert atmosphere.

The agitated solution A was charged with solution B, while maintaining the internal temperature below -25°C. The reaction mixture was agitated on -40°C for 60 minutes. After an hour the reaction mixture was quenched on a pre-cooled (-10°C) solution of KOH (12.6g) in water (42 L). The bi-phasic mixture was warmed to room temperature and the phases were separated. The organic layer was washed three times with brine (3x18 kg) and twice with water (2x18 L). The organic phase was concentrated to about 18 L. To the concentrated solution DCM (32.6 kg) was charged and it was concentrated to about 18 L where the concentrated solution was filtered. To the concentrated solution Toluene (50.8 kg) was added and it was concentrated to 39 L then the mixture was heated to 90°C for 10 minutes and it was cooled to 55°C over 60 minutes. The solution was cooled to 22°C over 120 minutes. The resulting suspension was filtered, washed with Toluene and was dried to dryness giving white crystals (1.9 kg, 62.3%).

### Example 36

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Under nitrogen to a mixture of 10.00 g (40.0 mmol) *tert*-butyl(dimethyl)silyl-N-4-Aminopyrrolo[2,1-*f*][1,2,4]triazine in 100 mL dry 2-Methyltetrahydrofuran was cooled in dry ice/ethanol bath. 33.5 mL of 2.5 M n-Hexyllithium solution in hexanes (83.7 mmol) was added dropwise at -70 - -60 °C. After addition the mixture was stirred at -70 °C for 0.5 h. To this mixture, a solution of 14.00 g (33.45 mmol) of (3*R*,4*R*,5*R*)-3,4-bis(benzyloxy)-5-[(benzyloxy)methyl]oxolan-2-one in 30 mL of dry 2-Methyltetrahydrofuran was added dropwise at -70 °C. After addition the mixture was stirred at -70 °C for 0.5 h. The reaction mixture was added into a mixture of 16.5 mL acetic acid and 56 mL water. After the quenching the mixture was stirred at 55-60 °C for 1 h. The mixture was cooled to room temperature. The phases were separated. The organic phase was washed 4 times with 50 mL 5% solution of citric acid, and twice with 50 mL saturated NaHCO₃ solution and concentrated *in vacuo* to 36 g residual solution. 70 mL of 2-butanone was added to the residual and the solution was concentrated *in vacuo* to 35 g residual. The solution was stirred at room temperature. 60 mL of hexanes was added to the solution. The mixture was stirred for 16 hours. Additional 10 mL of hexanes was added dropwise and the suspension was stirred for 1 hour at room temperature. The mixture was cooled to 0-5 °C, filtered and washed with 3x3 mL of 2-butanone:hexanes (1:4) mixture and with 3x3 mL of hexanes. The crystalls were dried under vacuum at 30 °C.
The mother liqour was evaporated. The residual was 10.14 g oil. The residual contained 4.60% (466 mg) of the title product (measured by HPLC, 2.5% of the theoretical yield)
Theoretical yield: 18.5 g
Isolated yield: 10.94 g white crystalls (59%)
Total yield: 11.406 g (61.5%)

### Example 37

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Under nitrogen to a mixture of 10.00 g (40.0 mmol) *tert*-butyl(dimethyl)silyl-N-4-Aminopyrrolo[2,1-*f*][1,2,4]triazine and 6.00 mL (5.664 g, 42.2 mmol) of dry diglyme in 100 mL dry 2-Methyltetrahydrofuran was cooled in dry ice/ethanol bath. 33.5 mL of 2.5 M n-Hexyllithium solution in hexanes (83.7 mmol) was added dropwise at -70 - -60 °C. After addition the mixture was stirred at -70 °C for 0.5 h. To this mixture, a solution of 14.00 g (33.45 mmol) (3*R*,4*R*,5*R*)-3,4-bis(benzyloxy)-5-[(benzyloxy)methyl]oxolan-2-one in 30 mL of dry 2-Methyltetrahydrofuran was added dropwise at -70 °C. After addition the mixture was stirred at -70 °C for 0.5 h. The reaction mixture was added into a mixture of 16.5 mL acetic acid and 56 mL water. After the quenching the mixture was stirred at 55-60 °C for 1 h. The mixture was cooled to room temperature. The phases were separated. The organic phase was washed 4 times with 50 mL 5% solution of citric acid, and twice with 50 mL saturated NaHCO₃ solution and concentrated *in vacuo* to 40 g residual solution. 70 mL 2-butanone was added to the residual and the solution was concentrated *in vacuo* to 40 g residual. The solution was stirred at room temperature. 25 mL hexanes was added to the solution. The mixture was seeded with (3*R*,4*R*,5*R*)-2-(4-aminopyrrolo[2,1-*f*][1,2,4]triazin-7-yl)-3,4-bis(benzyloxy)-5-((benzyloxy)methyl)tetrahydrofuran-2-ol (prepared according to Example 36) and was stirred for 60 minutes. Additional 50 mL of hexanes was added dropwise and the suspension was stirred for 1 hour at room temperature. The mixture was cooled to 0-5 °C, filtered and washed with 4x6 mL 2-butanone:hexanes (1:4) mixture. The crystalls were dried under vacuum at 30 °C.
The mother liqour was evaporated. The residual was 7.425 g oil. The residual contained 4.64% (345 mg) of the title product (measured by HPLC, 1.9% of the theoretical yield)
Theoretical yield: 18.5 g
Isolated yield: 14.47 g white crystalls (78.2%)
Total yield: 14.815 g (80.1%)

### Example 38

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Under nitrogen to a mixture of 10.00 g (40.0 mmol) *tert*-butyl(dimethyl)silyl-N-4-Aminopyrrolo[2,1-*f*][1,2,4]triazine and 6.00 mL (5.92 g, 33.2 mmol) of dry triglyme in 100 mL of dry 2-Methyltetrahydrofuran was cooled in dry ice/ethanol bath. 33.5 mL of 2.5 M n-Hexyllithium solution in hexanes (83.7 mmol) was added dropwise at -70 - -60 °C. After addition the mixture was stirred at -70 °C for 0.5 h. To this mixture, a solution of 14.00 g (33.45 mmol) (3*R*,4*R*,5*R*)-3,4-bis(benzyloxy)-5-[(benzyloxy)methyl]oxolan-2-one in 30 mL of dry 2-Methyltetrahydrofuran was added dropwise at -70 °C. After addition the mixture was stirred at -70 °C for 0.5 h. The reaction mixture was added into a mixture of 16.5 mL acetic acid and 56 mL water. After the quenching the mixture was stirred at 55-60 °C for 1 h. The mixture was cooled to room temperature. The phases were separated. The organic phase was washed 4 times with 50 mL 5% solution of citric acid, and twice with 50 mL saturated NaHCO₃ solution and concentrated *in vacuo* to approx. 20 g residual. 20 mL of 2-butanone was added to solve the crude product. The solution was stirred at room temperature. 22 mL of hexanes was added to the solution. The mixture was seeded with (3*R*,4*R*,5*R*)-2-(4-aminopyrrolo[2,1-*f*][1,2,4]triazin-7-yl)-3,4-bis(benzyloxy)-5-((benzyloxy)methyl)tetrahydrofuran-2-ol (prepared according to Example 36) and was stirred for 60 minutes. Additional 58 mL of hexanes was added dropwise and the suspension was stirred for 16 hours at room temperature. The mixture was cooled to 0-5 °C, filtered and washed with 4x5 mL of 2-butanone:hexanes (1:4) mixture. The crystalls were dried under vacuum at 30 °C.
Theoretical yield: 18.5 g
Yield: 13.58 g white crystalls (73%)

### Example 39

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Under nitrogen to a mixture of 5.00 g (20.1 mmol) *tert*-butyl(dimethyl)silyl-N-4-Aminopyrrolo[2,1-*f*][1,2,4]triazine and 4.60 mL (4.30 g, 32 mmol) of dry tetraglyme in 50 mL of dry 2-Methyltetrahydrofuran was cooled in dry ice/acetonitrile bath (approx. -40 °C). 17 mL of 2.5 M n-Hexyllithium solution in hexanes (42.0 mmol) was added dropwise at -40 °C. After addition the mixture was stirred at -40 °C for 0.5 h. To this mixture, a solution of 7.00 g (16.7 mmol) (3*R*,4*R*,5*R*)-3,4-bis(benzyloxy)-5-[(benzyloxy)methyl]oxolan-2-one in 15 mL of dry 2-Methyltetrahydrofuran was added dropwise at -40°C. After addition the mixture was stirred at -40°C for 0.5 h. The reaction mixture was added into a mixture of 8.25 mL acetic acid and 28 mL water. After the quenching the mixture was stirred at 55-60 °C for 1 h. The mixture was cooled to room temperature. The phases were separated. The organic phase was washed 4 times with 50 mL 5% solution of citric acid, and with 50 mL saturated NaHCO₃ solution and concentrated *in vacuo* to approx. 20 g residual. 20 mL of 2-butanone was added to solve the crude product. The solution was stirred at room temperature. 10 mL of hexanes was added to the solution. The mixture was seeded with (3*R*,4*R*,5*R*)-2-(4-aminopyrrolo[2,1-*f*][1,2,4]triazin-7-yl)-3,4-bis(benzyloxy)-5-((benzyloxy)methyl)tetrahydrofuran-2-ol (prepared according to Example 36) and was stirred for 60 minutes. Additional 28 mL of hexanes was added dropwise and the suspension was stirred for 16 hours at room temperature. The mixture was cooled to 0-5 °C, filtered and washed with 4x2.5 mL of 2-butanone:hexanes (1:4) mixture. The crystalls were dried under vacuum at 30 °C.
Theoretical yield: 9.23 g
Yield: 6.53 g white crystalls (70%)

### Example 40

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Under nitrogen to a mixture of 5.85 g (20.2 mmol) tri(isopropyl)silyl-N-4-Aminopyrrolo[2,1-*f*][1,2,4]triazine and 3.00 mL (2.81 g, 21.0 mmol) of dry diglyme in 60 mL of dry 2-Methyltetrahydrofuran was cooled in dry ice/ethanol bath. 17.0 mL of 2.5 M n-Hexyllithium solution in hexanes (42 mmol) was added dropwise at -70 - -60 °C. After addition the mixture was stirred at -70 °C for 0.5 h. To this mixture, a solution of 7.00 g (16.7 mmol) (3*R*,4*R*,5*R*)-3,4-bis(benzyloxy)-5-[(benzyloxy)methyl]oxolan-2-one in 15 mL of dry 2-Methyltetrahydrofuran was added dropwise at -70 °C. After addition the mixture was stirred at -70 °C for 0.5 h. The reaction mixture was added into 100 mL 10% citric acid solution. After 10 minutes stirring the phases were separated. 2.25 g (8.06 mmol) of tetrabutylammonium fluoride hydrate was added to the organic phase. The reaction mixture was stirred at room temperature for one hour. The mixture was washed 4 times with 25 mL 5% solution of citric acid, and with 25 mL saturated NaHCO₃ solution and concentrated *in vacuo* to 16.5 g residual. 15 mL of 2-butanone was added to the residual and the solution was concentrated *in vacuo* to 14.8 g residual. The residual was dissolved in 10 mL 2-butanone. The solution was stirred at room temperature. 40 mL of hexanes was added dropwise to the solution. The mixture was seeded with (3*R*,4*R*,5*R*)-2-(4-aminopyrrolo[2,1-*f*][1,2,4]triazin-7-yl)-3,4-bis(benzyloxy)-5-((benzyloxy)methyl)tetrahydrofuran-2-ol (prepared according to Example 36) and was stirred for 60 minutes. The mixture was cooled to 0-5 °C, filtered and washed with 4x3 mL of 2-butanone:hexanes (1:4) mixture. The crystalls were dried under vacuum at 30 °C.
Theoretical yield: 9.23 g
Yield: 6.35 g (69%) off-white crystalls

### Example 41

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Under nitrogen to a mixture of 5.00 g (13.5 mmol) 7-bromo-tri(isopropyl)silyl-N-4-Aminopyrrolo[2,1-*f*][1,2,4]triazine and 2.00 mL (1.87 g, 14.0 mmol) of dry diglyme in 50 mL of dry 2-Methyltetrahydrofuran was cooled in dry ice/ethanol bath. 12.5 mL of 2.5 M n-Hexyllithium solution in hexanes (29 mmol) was added dropwise at -70 - -60 °C. After addition the mixture was stirred at -70 °C for 0.5 h. To this mixture, a solution of 4.53 g (10.8 mmol) (3*R*,4*R*,5*R*)-3,4-bis(benzyloxy)-5-[(benzyloxy)methyl]oxolan-2-one in 10 mL of dry 2-Methyltetrahydrofuran was added dropwise at -70 °C. After addition the mixture was stirred at -70 °C for 0.5 h. The reaction mixture was added into a acetic acid solution (6.0 mL acetic acid and 20 mL water). After 60 minutes stirring the phases were separated. The organic phase was washed three times with 25 mL saturated NaHCO₃ solution. 2.14 g (6.77 mmol) of tetrabutylammonium fluoride hydrate was added to the organic phase. The reaction mixture was stirred at room temperature for one hour. The mixture was washed 4 times with 35 mL 5% solution of citric acid, and with 35 mL of saturated NaHCO₃ solution and concentrated *in vacuo* to 15.3 g residual. 30 mL of 2-butanone was added to the residual and the solution was concentrated *in vacuo* to 15.3 g residual. The solution was stirred at room temperature. 10 mL of hexanes was added dropwise to the solution. The mixture was seeded with (3*R*,4*R*,5*R*)-2-(4-aminopyrrolo[2,1-*f*][1,2,4]triazin-7-yl)-3,4-bis(benzyloxy)-5-((benzyloxy)methyl)tetrahydrofuran-2-ol (prepared according to Example 36). Additional 20 mL of hexanes was added dropwise and was stirred for 10 minutes at room temperature. The mixture was cooled to 0-5 °C, filtered and washed with 4x2.5 mL 2-butanone:hexanes (1:4) mixture. The crystalls were dried under vacuum at 30 °C.
Theoretical yield: 5.97 g
Yield: 3.55 g, 59% white crystalls

### Example 42

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Under nitrogen to a mixture of 75.00 g (301.9 mmol) *tert*-butyl(dimethyl)silyl-N-4-Aminopyrrolo[2,1-*f*][1,2,4]triazine and 45.0 mL (42.2 g, 314 mmol) of dry diglyme in 750 mL dry 2-Methyltetrahydrofuran was cooled to -70°C. 250 mL of 2.5 M n-Hexyllithium solution in hexanes (620 mmol) was added dropwise at -70 - -58 °C. After addition the mixture was stirred at -70 °C for 0.5 h. To this mixture, a solution of 105.0 g (250.9 mmol) (3*R*,4*R*,5*R*)-3,4-bis(benzyloxy)-5-[(benzyloxy)methyl]oxolan-2-one in 210 mL of dry 2-Methyltetrahydrofuran was added dropwise at -71 - -56 °C. After addition the mixture was stirred at -70 °C for 0.5 h. The reaction mixture was added into a mixture of 125 mL acetic acid and 420 mL water. After the quenching the mixture was stirred at 55-60 °C for 1 h. The mixture was cooled to room temperature. The phases were separated. The organic phase was washed 4 times with 375 mL 5% solution of citric acid, and twice with 375 mL saturated NaHCO₃ solution and concentrated *in vacuo* to 300 mL residual solution. 525 mL 2-butanone was added to the residual and the solution was concentrated *in vacuo* to 285 mL residual. The solution was stirred at room temperature. 110 mL hexanes was added to the solution. The mixture was seeded with (3*R*,4*R*,5*R*)-2-(4-aminopyrrolo[2,1-*f*][1,2,4]triazin-7-yl)-3,4-bis(benzyloxy)-5-((benzyloxy)methyl)tetrahydrofuran-2-ol (prepared according to Example 36) and was stirred for 60 minutes. Additional 450 mL of hexanes was added dropwise and the suspension was stirred for 1 hour at room temperature. The mixture was cooled to 0-5 °C, filtered and washed with 4x45 mL 2-butanone:hexanes (1:4) mixture. The crystalls were dried under vacuum at 30 °C.
Theoretical yield: 138.7 g
Isolated yield: 104.7 g white crystalls (75.5%)

### NMR SPECTROSCOPIC CHARACTERIZATION

| | |
|---|---|
| *Instrument:* | BRUKER-800 NMR spectrometer (¹H: 799.7 MHz, ¹³C: 201.1 MHz) |
| *Solvent:* | DMSO-*d₆* |
| *Temperature:* | 25 °C |
| *Reference:* | δ_{TMS} = 0.00 ppm (¹H), δ_{TMS} = 0.00 (¹³C) |

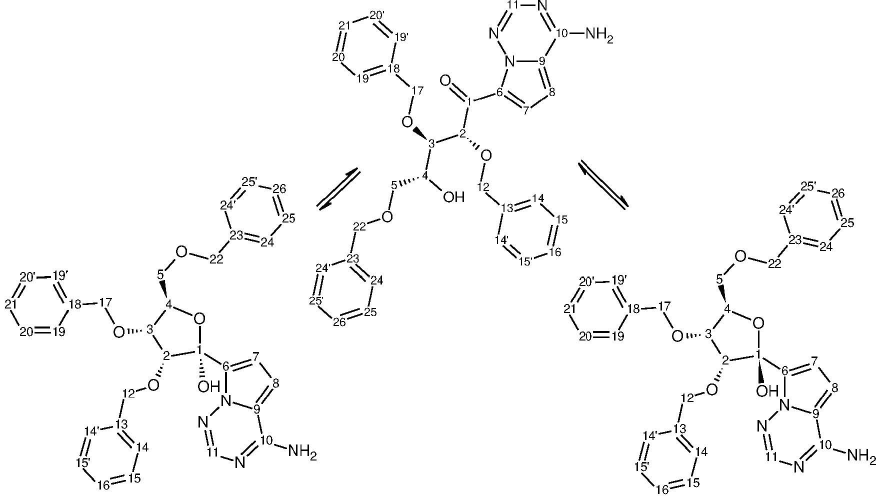

The major isomer is the open chain form in DMSO-*d₆* solution, the α-OH furanose and β-OH furanose are present in 0.07 and 0.09 molar ratio. The relative configuration was proven by through space 2D ROESY correlations in furanose forms.

| **Open chain form** | **¹H NMR, δ (ppm)** | **¹³C NMR, δ (ppm)** |
|---|---|---|
| C-1 | - | 187.8 |
| CH-2 | 5.39 d ³*J*_{HH}=6.0 Hz | 80.7 |
| CH-3 | 3.93 dd ³*J*_{HH}=6.0, 4.5 Hz | 81.8 |
| CH-4 | 4.02 m | 69.2 |
| OH | 5.07 d ³*J*_{HH}=5.3 Hz | - |
| CH₂-5 | 3.48 dd ²*J*_{HH}=10.0 Hz ³*J*_{HH}=6.5 Hz & 3.69 dd ²*J*_{HH}=10.0 Hz ³*J*_{HH}=3.5 | 71.6 |
| C-6 | - | 128.5 |
| CH-7 | 7.34 d ³*J*_{HH}=4.8 Hz | 117.4 |
| CH-8 | 6.95 d ³*J*_{HH}=4.8 Hz | 102.1 |
| C-9 | - | 118.4 |
| C-10 | - | 155.7 |
| NH₂ | 8.06 br & 8.09 br | - |
| CH-11 | 8.00 s | 148.9 |
| CH₂-12 | 4.47 m & 4.57 m | 71.3 |
| C-13 | - | 138.0 |
| CH-14, CH-14', CH-24, CH-24' | 7.28-7.33 m | 127.3, 127.4 |
| CH-15, CH-15', CH-25, CH-25' | 7.28-7.33 m | 128.03, 128.04 |
| CH-16, CH-26 | 7.27 m | 127.2, 127.4 |
| CH₂-17 | 4.49 m & 4.57 m | 72.33 |
| C-18 | - | 138.3 |
| CH-19, CH-19' | 7.00 m | 127.1 |
| CH-20, CH-20' | 7.15 m | 127.7 |
| CH-21 | 7.16 m | 127.0 |
| CH₂-22 | 4.47 m & 4.56 m | 72.2 |
| **α-OH furanose form** | ¹H NMR, δ (ppm) | ¹³C NMR, δ (ppm) |
| C-1 | - | 100.5 |
| OH | 6.29 s | - |
| CH-2 | 4.64 m | 77.0 |
| CH-3 | 3.97 t ³*J*_{HH}=6.0 Hz | 76.9 |
| CH-4 | 4.30 m | 78.8 |
| CH₂-5 | 3.58 dd ²*J*_{HH}=10.7 Hz ³*J*_{HH}=5.8 Hz & 3.67 m | 69.9 |
| C-6 | - | 130.0 |
| CH-7 | 6.72 d ³*J*_{HH}=4.4 Hz | 110.1 |
| CH-8 | 6.82 d ³*J*_{HH}=4.4 Hz | 100.0 |
| C-9 | - | 115.3 |
| C-10 | - | 155.52 |
| NH₂ | 7.66 br | - |
| CH-11 | 7.74 s | 147.1 |
| CH₂-12 | 4.61 m & 4.72 m | 71.7 |
| C-13, C-18, C-23 | - | 138.07, 138.15 |
| CH-14, CH-14', CH-15, CH-15', CH-16, CH-19, CH-19', CH-20, CH-20', CH-21, CH-24, CH-24', CH-25, CH-25', CH-26 | 7.13-7.37 m | 127.02, 127.28, 127.30, 127.48, 127.52, 127.75, 127.86, 128.08 |
| CH₂-17 | 4.42 m & 4.48 m | 71.2 |
| CH₂-22 | 4.47 m & 4.56 m | 72.14 |
| **β-OH furanose form** | ¹H NMR, δ (ppm) | ¹³C NMR, δ (ppm) |
| C-1 | - | 103.0 |
| OH | 6.69 s | - |
| CH-2 | 4.61 m | 80.4 |
| CH-3 | 4.31 m | 80.1 |
| CH-4 | 4.16 m | 78.7 |
| CH₂-5 | 3.61 dd ²*J*_{HH}=10.6 Hz ³*J*_{HH}=6.8 Hz & 3.66 m | 72.5 |
| C-6 | - | 129.8 |
| CH-7 | 6.68 d ³*J*_{HH}=4.4 Hz | 110.2 |
| CH-8 | 6.84 d ³*J*_{HH}=4.4 Hz | 100.0 |
| C-9 | - | 114.6 |
| C-10 | - | 155.47 |
| NH₂ | 7.66 br | - |
| CH-11 | 7.81 s | 147.2 |
| CH₂-12 | 4.08 m & 4.39 m | 72.35 |
| C-13, C-18, C-23 | - | 138.11, 138.33 |
| CH-14, CH-14' | 6.67 m | 126.90 |
| CH-15, CH-15' | 7.08 m | 127.65 |
| CH-16 | 7.12 m | 127.09 |
| CH-19, CH-19', CH-20, CH-20', CH-21, CH-24, CH-24', CH-25, CH-25', CH-26 | 7.13-7.37 m | 127.50, 127.61, 127.65, 128.11, 128.14 |
| CH₂-17 | 4.54 m & 4.64 m | 71.6 |
| CH₂-22 | 4.53 m | 72.11 |

### Example 43

### (3R,4R,5R)-2-(4-aminopyrrolo[2,1-f][1,2,4]triazin-7-yl)-3,4-bis(benzyloxy)-5-((benzyloxy)methyl)tetrahydrofuran-2-ol

Under nitrogen to a mixture of 5.00 g (20.1 mmol) *tert*-butyl(dimethyl)silyl-N-4-Aminopyrrolo[2,1-*f*][1,2,4]triazine and 4.45 mL (4.49 g, 20.2 mmol) of dry tetraglyme in 50 mL of dry 2-Methyltetrahydrofuran was cooled in dry ice/ethanol bath (approx. -70 °C). 17 mL of 2.5 M n-Hexyllithium solution in hexanes (42.0 mmol) was added dropwise. After addition the mixture was stirred at -70 °C for 0.5 h. To this mixture, a solution of 7.00 g (16.7 mmol) (3*R*,4*R*,5*R*)-3,4-bis(benzyloxy)-5-[(benzyloxy)methyl]oxolan-2-one in 15 mL of dry 2-Methyltetrahydrofuran was added dropwise at -70- -60 °C. After addition the mixture was stirred at -70°C for 0.5 h. The reaction mixture was added into a mixture of 9.0 mL acetic acid and 28 mL water. After the quenching the mixture was stirred at 55-60 °C for 1 h. The mixture was cooled to room temperature. The phases were separated. The organic phase was washed 4 times with 30 mL 5% solution of citric acid, and twice with 30 mL saturated NaHCO₃ solution and concentrated *in vacuo* to approx. 20 g residual. 35 mL of 2-butanone was added to solve the crude product. The solution was evaporated to 20.1 g residual. The solution was stirred at room temperature. 7.3 mL of hexanes was added to the solution. The mixture was seeded with (3*R*,4*R*,5*R*)-2-(4-aminopyrrolo[2,1-*f*][1,2,4]triazin-7-yl)-3,4-bis(benzyloxy)-5-((benzyloxy)methyl)tetrahydrofuran-2-ol and was stirred for 60 minutes. Additional 30 mL of hexanes was added dropwise and the suspension was stirred for one hour at room temperature. The mixture was cooled to 0-5 °C, filtered and washed with 4x3.0 mL of 2-butanone:hexanes (1:4) mixture. The crystalls were dried under vacuum at 30 °C.
Theoretical yield: 9.23 g
Yield: 7.00 g white crystalls (76%)

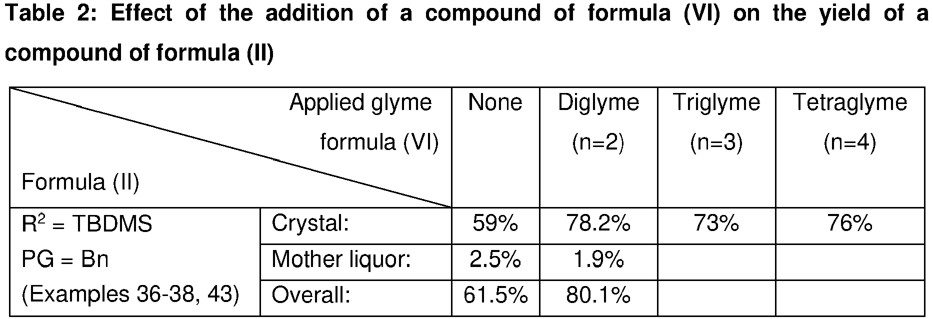

## Claims

1. A process for the preparation of a compound of formula (I) 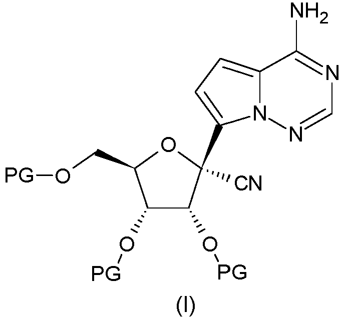 wherein
PG is a protecting group selected from the group consisting of trimethylsilyl, *t-*butyldimethylsilyl, *t*-butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a -C(R³)₂- group wherein R³ is H, or C₁₋₆alkylene group comprising cyanation of a compound of formula (II) 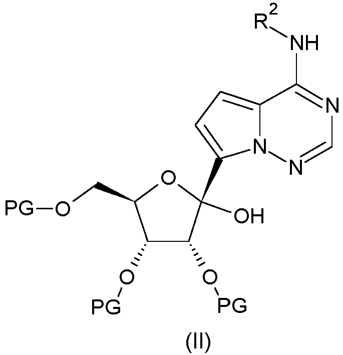 wherein
R² is hydrogen or a protecting group selected from the group consisting of *t-*butyldimethylsilyl, triisopropylsilyl and *t*-butyldiphenylsilyl, and
PG is a protecting group selected from the group consisting of trimethylsilyl, *t-*butyldimethylsilyl, *t*-butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a -C(R³)₂- group wherein R³ is H, or C₁₋₆alkylene group
**characterized in that**
the compound of formula (II) is obtained by the coupling reaction of a compound of formula (IV) 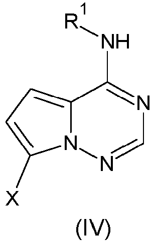 wherein
X is hydrogen, bromine or iodine, and
R¹ is a protecting group selected from the group consisting of *t*-butyldimethylsilyl, triisopropylsilyl and *t*-butyldiphenylsilyl
and a compound of formula (III) 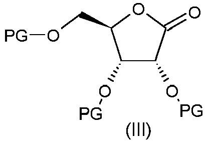 wherein
PG is a protecting group selected from the group consisting of trimethylsilyl, *t-*butyldimethylsilyl, *t*-butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a -C(R³)₂- group wherein R³ is H, or C₁₋₆alkylene group in the presence of a lithiation reagent.

2. The process according to claim 1, wherein the cyanation step is carried out in a suitable solvent and in the presence of a Brønsted acid with a solution of a Lewis acid and a cyanating agent.

3. The process according to any one of claims 1 or 2, wherein in the cyanation step the solvent is dichloromethane,
the Brønsted acid is trifluoro-acetic acid,
the Lewis acid is trimethylsilyl trifluoromethanesulfonate and
the cyanating agent is trimethylsilyl cyanide.

4. The process according to any one of claims 1 to 3,
- further comprising recrystallization of the compound of formula (I) from toluene, and/or
- wherein the compound of formula (IV) is solved in a cyclic ether, and/or
- wherein the lithiation reagent is an alkyllithium solved in a higher alkane, and/or
- wherein the compounds of formula (II) are isolated or wherein in the compounds of formula (II) R² is a protecting group are reacted further without isolation, preferably further comprising deprotection of the compounds of formula (II) wherein R² is a protecting group using a cleaving agent to obtain a compound of formula (II) wherein R² is hydrogen, preferably wherein the cleaving agent is tetrabutylammonium fluoride trihydrate or acetic acid.

5. The process according to any one of claims 1 to 4, wherein in the compound of formula (II) R² is hydrogen.

6. The process according to claim 5, further comprising the purification of the compounds of formula (II) wherein R² is hydrogen.

7. The process according to any one of claims 1 to 6, wherein in the compounds of formula (III) PG is a protecting group is selected from the group consisting of t-butyldimethylsilyl and benzyl, or two protecting groups on adjacent carbon atoms can be combined to form a -C(R³)₂- group wherein R³ is H, or C₁₋₃alkylene group.

8. The process according to any one of claims 1 to 7,
- wherein in the compound of formula (III) PG is benzyl group; and/or
- wherein in the compounds of formula (IV) X is hydrogen or bromine and R¹ is a protecting group selected from the group consisting of t-butyldimethylsilyl and triisopropylsilyl; and/or
- wherein in the compounds of formula (IV) X is hydrogen and R¹ is t-butyldimethylsilyl; and/or
- wherein the compound of formula (III) is solved in toluene or a cyclic ether; and/or
- further comprising protecting a compound of formula (V) to obtain the compounds of formula (IV) 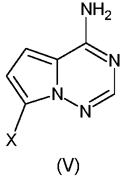 wherein
X is hydrogen, bromine or iodine
in a suitable solvent and in the presence of a suitable base by the addition of a protecting agent selected from the group consisting of *t*-butyldimethylsilyl chloride, triisopropylsilyl chloride and *t*-butyldiphenylsilyl chloride, preferably
- in the protection step the suitable solvent for a compound of formula (V) is a cyclic ether, and the suitable base is an alkyllithium, an alkali hydroxide or an alkali alkoxide, and/or
- the protection step further comprises recrystallization from a higher alkane, and/or
- in the compound of formula (V) X is hydrogen.

9. The process according to any one of claims 1 to 8, wherein the coupling reaction of a compound of formula (IV) and a compound of formula (III) is carried out in the presence of a compound of formula (VI) wherein n is 2, 3 or 4

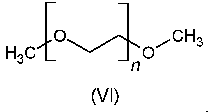

10. The process according to claim 9, wherein the compound of formula (VI) is solved in a cyclic ether, and/or wherein in the compounds of formula (VI) n is 2 or 3.

11. The process according to claim 10, wherein in the compounds of formula (VI) n is 2.

12. The process according to any one of claims 1 to 8, wherein the coupling reaction is carried out in continuous flow method wherein a system of two sequential continuous-flow microreactors (1 and 2) is used comprising:
- a microreactor 1 comprising a micromixer and a coil reactor for mixing of the compound of formula (IV) with the lithiation reagent and reacting them for a residence time of 20 to 45 sec to obtain a corresponding lithiated intermediate,
- a microreactor 2 comprising a micromixer and a coil reactor for mixing said lithiated intermediate with a compound of formula (III) and reacting them for a residence time of 70 to 150 sec to obtain the compound of formula (II),
- input storages, pumps and injector modules providing three distinct inlets A, B and C that are connected to the two sequential microreactors,
- pre-cooling loops for each inlet, and
- a thermostated bath,
wherein a solution of injector A, and a solutions of injector B (microreactor 1) and injector C (microreactor 2) are sequentially injected into a flowing stream of dry THF for pre-determined periods of time, followed by the collection of the mixture and an aqueous work-up thereof, and wherein
the solution of Injector A is a solution of a compound of formula (IV),
the solution of injector B is a solution of a lithiation reagent, and
the solution of injector C is a solution of a compound of formula (III).

13. The process according to claim 12, wherein the microreactors 1 and 2 and pre-cooling loops for each inlet are thermostated at -30°C, and/or wherein the reaction temperature in the microreactors is -10 to -30°C.

14. A compound of formula (II) 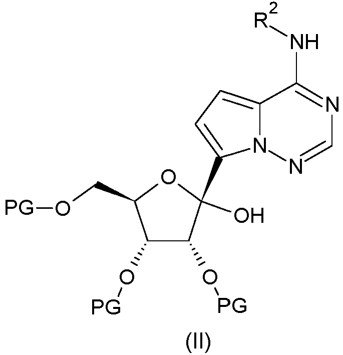 wherein
R² is a protecting group selected from the group consisting of *t*-butyldimethylsilyl, triisopropylsilyl and *t*-butyldiphenylsilyl, and
PG is a protecting group selected from the group consisting of trimethylsilyl, *t-*butyldimethylsilyl, *t*-butyldiphenylsilyl, methyl-methoxy, tetrahydropyran, *t*-butyl, allyl, benzyl, acetyl, pivaloyl and benzoyl, or two protecting groups on adjacent carbon atoms can be combined to form a -C(R³)₂- group wherein R³ is H, or C₁₋₆alkylene group.

15. A compound of formula (IV) 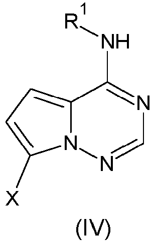 wherein
X is hydrogen, bromine or iodine, and
R¹ is a protecting group selected from the group consisting of *t*-butyldimethylsilyl, triisopropylsilyl and *t*-butyldiphenylsilyl.

## Patentansprüche

1. Ein Verfahren zur Herstellung einer Verbindung der Formel (I) 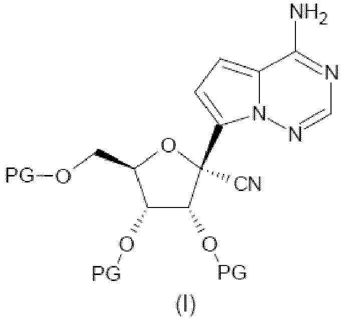 wobei
PG eine Schutzgruppe ist, die aus der Gruppe ausgewählt ist, die aus Trimethylsilyl, *t*-Butyldimethylsilyl, t-Butyldiphenylsilyl, Methyl-methoxy, Tetrahydropyran, *t*-Butyl, Allyl, Benzyl, Acetyl, Pivaloyl und Benzoyl besteht, oder zwei Schutzgruppen an benachbarten Kohlenstoffatomen kombiniert werden können, um eine -C(R³)₂-Gruppe zu bilden, worin R³ H oder eine C₁₋₆-Alkylengruppe ist, umfassend die Cyanierung einer Verbindung der Formel (II) 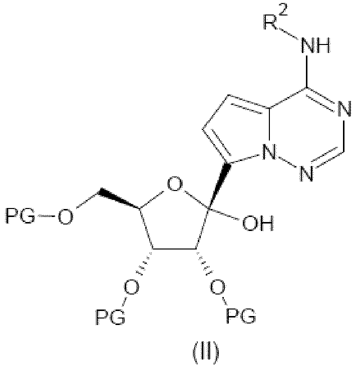 wobei
R² Wasserstoff oder eine Schutzgruppe ist, ausgewählt aus der Gruppe bestehend aus *t*-Butyldimethylsilyl, Triisopropylsilyl und *t*-Butyldiphenylsilyl, und
PG eine Schutzgruppe ist, die aus der Gruppe ausgewählt ist, die aus Trimethylsilyl, *t*-Butyldimethylsilyl, *t*-Butyldiphenylsilyl, Methyl-methoxy, Tetrahydropyran, *t*-Butyl, Allyl, Benzyl, Acetyl, Pivaloyl und Benzoyl besteht, oder zwei Schutzgruppen an benachbarten Kohlenstoffatomen kombiniert werden können, um eine -C(R³)₂-Gruppe zu bilden, worin R³ H oder eine C₁₋₆-AlkylenGruppe ist
**dadurch gekennzeichnet, dass**
die Verbindung der Formel (II) erhalten wird durch die Kupplungsreaktion einer Verbindung der Formel (IV) 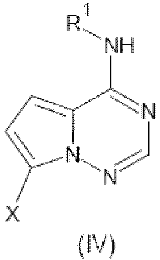 wobei
X Wasserstoff, Brom oder Jod ist, und
R¹ eine Schutzgruppe ist, ausgewählt aus der Gruppe bestehend aus *t-*Butyldimethylsilyl, Triisopropylsilyl und *t*-Butyldiphenylsilyl
und eine Verbindung der Formel (III) 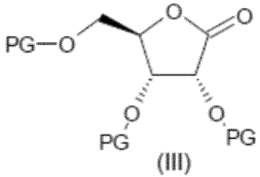 wobei
PG eine Schutzgruppe ist, die aus der Gruppe ausgewählt ist, die aus Trimethylsilyl, *t*-Butyldimethylsilyl, *t*-Butyldiphenylsilyl, Methyl-Methoxy, Tetrahydropyran, *t*-Butyl, Allyl, Benzyl, Acetyl, Pivaloyl und Benzoyl besteht, oder zwei Schutzgruppen an benachbarten Kohlenstoffatomen kombiniert werden können, um eine -C(R³)₂-Gruppe zu bilden, worin R³ H oder eine C₁₋₆-AlkylenGruppe ist
in Anwesenheit eines Lithiierungsreagenzes.

2. Das Verfahren nach Anspruch 1, wobei die Cyanisierungsstufe in einem geeigneten Lösungsmittel und in Gegenwart einer Brønsted-Säure mit einer Lösung einer Lewis-Säure und einem Cyanisierungsmittel durchgeführt wird.

3. Das Verfahren nach einem der Ansprüche 1 oder 2, wobei in der Cyanisierungsstufe das Lösungsmittel Dichlormethan ist,
die Brønsted-Säure Trifluoressigsäure ist,
die Lewis-Säure Trimethylsilyltrifluormethansulfonat ist und
das Cyanisierungsmittel Trimethylsilylcyanid ist.

4. Das Verfahren nach einem der Ansprüche 1 bis 3,
- weiter umfassend die Umkristallisation der Verbindung der Formel (I) aus Toluol, und/oder
- wobei die Verbindung der Formel (IV) in einem zyklischen Ether gelöst ist, und/oder
- wobei das Lithiierungsreagenz ein Alkyllithium ist, das in einem höheren Alkan gelöst ist, und/oder
- wobei die Verbindungen der Formel (II) isoliert werden oder wobei in den Verbindungen der Formel (II) R² eine Schutzgruppe ist, ohne Isolierung weiter umgesetzt werden, vorzugsweise weiter umfassend die Entschützung der Verbindungen der Formel (II), wobei R² eine Schutzgruppe ist, unter Verwendung eines Spaltmittels, um eine Verbindung der Formel (II) zu erhalten, wobei R² Wasserstoff ist, vorzugsweise wobei das Spaltmittel Tetrabutylammoniumfluoridtrihydrat oder Essigsäure ist.

5. Das Verfahren nach einem der Ansprüche 1 bis 4, wobei in der Verbindung der Formel (II) R² Wasserstoff ist.

6. Das Verfahren nach Anspruch 5, ferner umfassend die Reinigung der Verbindungen der Formel (II) worin R² Wasserstoff ist.

7. Das Verfahren nach einem der Ansprüche 1 bis 6, wobei in den Verbindungen der Formel (III) PG eine Schutzgruppe ist, ausgewählt aus der Gruppe bestehend aus *t*-Butyldimethylsilyl und Benzyl, oder zwei Schutzgruppen an benachbarten Kohlenstoffatomen kombiniert werden können, um eine -C(R³)₂-Gruppe zu bilden, worin R³ H oder eine C₁₋₃-Alkylengruppe ist.

8. Das Verfahren nach einem der Ansprüche 1 bis 7,
- wobei in der Verbindung der Formel (III) PG eine Benzylgruppe ist; und/oder
- wobei in den Verbindungen der Formel (IV) X Wasserstoff oder Brom ist und R¹ eine Schutzgruppe, ausgewählt aus der Gruppe bestehend aus *t-*Butyldimethylsilyl und Triisopropylsilyl, ist; und/oder
- wobei in den Verbindungen der Formel (IV) X Wasserstoff ist und R¹ *t-*Butyldimethylsilyl ist; und/oder
- worin die Verbindung der Formel (III) in Toluol oder einem cyclischen Ether gelöst ist; und/oder
- ferner das Schützen einer Verbindung der Formel (V) umfassend, um die Verbindungen der Formel (IV) zu erhalten 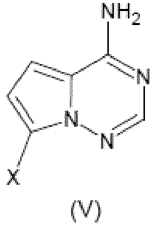 wobei
X Wasserstoff, Brom oder Jod ist
in einem geeigneten Lösungsmittel und in Gegenwart einer geeigneten Base durch Zugabe eines Schutzmittels, ausgewählt aus der Gruppe bestehend aus *t*-Butyldimethylsilylchlorid, Triisopropylsilylchlorid und *t*-Butyldiphenylsilylchlorid, vorzugsweise
- ist in der Schutzstufe das geeignete Lösungsmittel für eine Verbindung der Formel (V) ein cyclischer Ether und die geeignete Base ein Alkyllithium, ein Alkalihydroxid oder ein Alkalialkoxid, und/oder
- umfasst der Schutzschritt außerdem die Umkristallisation aus einem höheren Alkan, und/oder
- ist in der Verbindung der Formel (V) X Wasserstoff.

9. Das Verfahren nach einem der Ansprüche 1 bis 8, wobei die Kupplungsreaktion einer Verbindung der Formel (IV) und einer Verbindung der Formel (III) in Gegenwart einer Verbindung der Formel (VI) durchgeführt wird, wobei n 2, 3 oder 4 ist

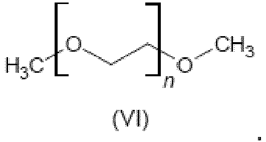

10. Das Verfahren nach Anspruch 9, wobei die Verbindung der Formel (VI) in einem cyclischen Ether gelöst ist und/oder wobei in den Verbindungen der Formel (VI) n 2 oder 3 ist.

11. Das Verfahren nach Anspruch 10, wobei in den Verbindungen der Formel (VI) n 2 ist.

12. Das Verfahren nach einem der Ansprüche 1 bis 8, bei dem die Kupplungsreaktion im Durchflussverfahren durchgeführt wird, wobei ein System aus zwei aufeinanderfolgenden Durchfluss-Mikroreaktoren (1 und 2) verwendet wird, das Folgendes umfasst:
- einen Mikroreaktor 1, der einen Mikromischer und einen Spulenreaktor umfasst, um die Verbindung der Formel (IV) mit dem Lithiierungsreagenz zu mischen und sie während einer Verweilzeit von 20 bis 45 Sekunden umzusetzen, um ein entsprechendes lithiiertes Zwischenprodukt zu erhalten,
- einen Mikroreaktor 2, der einen Mikromischer und einen Spulenreaktor umfasst, um das lithiierte Zwischenprodukt mit einer Verbindung der Formel (III) zu mischen und sie während einer Verweilzeit von 70 bis 150 Sekunden reagieren zu lassen, um die Verbindung der Formel (II) zu erhalten,
- Eingangsspeicher, Pumpen und Injektormodule mit drei verschiedenen Einlässen A, B und C, die mit den beiden sequenziellen Mikroreaktoren verbunden sind,
- Vorkühlschleifen für jeden Einlass, und
- ein thermostatisiertes Bad,
wobei eine Lösung von Injektor A und eine Lösung von Injektor B (Mikroreaktor 1) und Injektor C (Mikroreaktor 2) nacheinander in einen fließenden Strom von trockenem THF für vorbestimmte Zeiträume eingespritzt werden, gefolgt von der Sammlung der Mischung und einer wässrigen Aufarbeitung derselben, und wobei die Lösung von Injektor A eine Lösung einer Verbindung der Formel (IV) ist,
die Lösung von Injektor B eine Lösung eines Lithiierungsreagenzes ist, und
die Lösung des Injektors C eine Lösung einer Verbindung der Formel (III) ist.

13. Das Verfahren nach Anspruch 12, wobei die Mikroreaktoren 1 und 2 und die Vorkühlschleifen für jeden Einlass auf -30°C thermostatisiert sind und/oder wobei die Reaktionstemperatur in den Mikroreaktoren -10 bis -30°C beträgt.

14. Eine Verbindung der Formel (II) 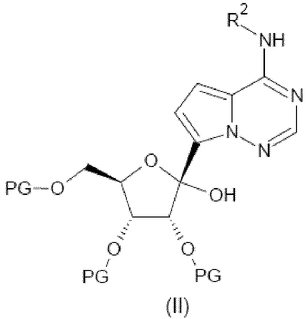 wobei
R² eine Schutzgruppe ist, ausgewählt aus der Gruppe bestehend aus *t-*Butyldimethylsilyl, Triisopropylsilyl und *t*-Butyldiphenylsilyl, und
PG eine Schutzgruppe ist, ausgewählt aus der Gruppe bestehend aus Trimethylsilyl, *t*-Butyldimethylsilyl, *t*-Butyldiphenylsilyl, Methyl-Methoxy, Tetrahydropyran, *t*-Butyl, Allyl, Benzyl, Acetyl, Pivaloyl und Benzoyl, oder zwei Schutzgruppen an benachbarten Kohlenstoffatomen kombiniert werden können, um eine -C(R³)₂- Gruppe zu bilden, wobei R³ H oder eine C₁₋₆-Alkylengruppe ist.

15. Eine Verbindung der Formel (IV) 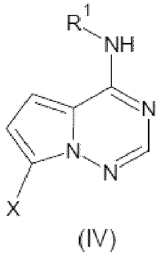 wobei
X Wasserstoff, Brom oder Jod ist, und
R¹ eine Schutzgruppe ist, ausgewählt aus der Gruppe bestehend aus *t-*Butyldimethylsilyl, Triisopropylsilyl und t-Butyldiphenylsilyl.

## Revendications

1. Procédé de préparation d'un composé de formule (I) 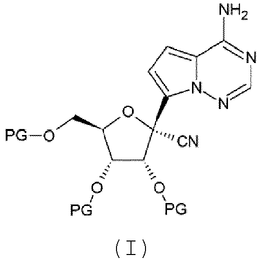 dans lequel
PG est un groupe protecteur choisi dans le groupe constitué par le triméthylsilyle, le *t*-butyldiméthylsilyle, le *t-*butyldiphénylsilyle, le méthylméthoxy, le tétrahydropyrane, le *t*-butyle, l'allyle, le benzyle, l'acétyle, le pivaloyle et le benzoyle, ou deux groupes protecteurs sur des atomes de carbone adjacents peuvent être combinés pour former un groupe -C(R³)₂-dans lequel R³ est H, ou un groupe alkylène en C₁₋₆ comprenant la cyanation d'un composé de formule (II) 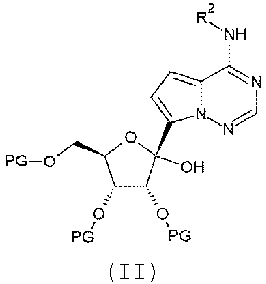 dans lequel
R² est hydrogène ou un groupe protecteur choisi dans le groupe constitué par le *t*-butyldiméthylsilyle, le triisopropylsilyle et le *t*-butyldiphénylsilyle, et
PG est un groupe protecteur choisi dans le groupe constitué par le triméthylsilyle, le *t*-butyldiméthylsilyle, le *t-*butyldiphénylsilyle, le méthylméthoxy, le tétrahydropyrane, le t-butyle, l'allyle, le benzyle, l'acétyle, le pivaloyle et le benzoyle, ou deux groupes protecteurs sur des atomes de carbone adjacents peuvent être combinés pour former un groupe -C(R³)₂-dans lequel R³ est H, ou un groupe alkylène en C₁₋₆
**caractérisé en ce que**
le composé de formule (II) est obtenu par la réaction de couplage d'un composé de formule (IV) 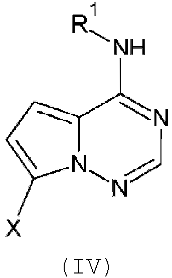 dans lequel
X est hydrogène, brome ou iode, et
R¹ est un groupe protecteur choisi dans le groupe constitué par le *t*-butyldiméthylsilyle, le triisopropylsilyle et le *t-*butyldiphénylsilyle
et un composé de formule (III) 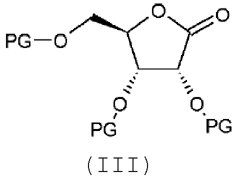 dans lequel
PG est un groupe protecteur choisi dans le groupe constitué par le triméthylsilyle, le *t*-butyldiméthylsilyle, le *t-*butyldiphénylsilyle, le méthylméthoxy, le tétrahydropyrane, le *t*-butyle, l'allyle, le benzyle, l'acétyle, le pivaloyle et le benzoyle, ou deux groupes protecteurs sur des atomes de carbone adjacents peuvent être combinés pour former un groupe -C(R³)₂-dans lequel R³ est H, ou un groupe alkylène en C₁₋₆ en présence d'un réactif de lithiation.

2. Procédé selon la revendication 1, dans lequel l'étape de cyanation est réalisée dans un solvant approprié et en présence d'un acide de Brønsted avec une solution d'un acide de Lewis et d'un agent de cyanation.

3. Procédé selon l'une quelconque des revendications 1 ou 2, dans lequel, dans l'étape de cyanation, le solvant est le dichlorométhane,
l'acide de Brønsted est l'acide trifluoroacétique,
l'acide de Lewis est le trifluorométhanesulfonate de triméthylsilyle et
l'agent de cyanation est le cyanure de triméthylsilyle.

4. Procédé selon l'une quelconque des revendications 1 à 3,
- comprenant également la recristallisation du composé de formule (I) à partir de toluène, et/ou
- dans lequel le composé de formule (IV) est dissous dans un éther cyclique, et/ou
- dans lequel le réactif de lithiation est un alkyllithium dissous dans un alcane supérieur, et/ou
- dans lequel les composés de formule (II) sont isolés ou dans lequel, dans les composés de formule (II), R² est un groupe protecteur sont mis à réagir davantage sans isolement, comprenant de préférence également la déprotection des composés de formule (II) dans lesquels R² est un groupe protecteur en utilisant un agent de clivage pour obtenir un composé de formule (II) dans lequel R² est hydrogène, de préférence dans lequel l'agent de clivage est le fluorure de tétrabutylammonium trihydraté ou l'acide acétique.

5. Procédé selon l'une quelconque des revendications 1 à 4, dans lequel, dans le composé de formule (II), R² est hydrogène.

6. Procédé selon la revendication 5, comprenant également la purification des composés de formule (II) dans lesquels R² est hydrogène.

7. Procédé selon l'une quelconque des revendications 1 à 6, dans lequel, dans les composés de formule (III), PG est un groupe protecteur choisi dans le groupe constitué par le *t-*butyldiméthylsilyle et le benzyle, ou deux groupes protecteurs sur des atomes de carbone adjacents peuvent être combinés pour former un groupe -C(R³)2- dans lequel R³ est H, ou un groupe alkylène en C₁₋₃.

8. Procédé selon l'une quelconque des revendications 1 à 7,
- dans lequel, dans le composé de formule (III), PG est un groupe benzyle ; et/ou
- dans lequel, dans les composés de formule (IV), X est hydrogène ou brome et R¹ est un groupe protecteur choisi dans le groupe constitué par le *t*-butyldiméthylsilyle et le triisopropylsilyle ; et/ou
- dans lequel, dans les composés de formule (IV), X est hydrogène et R¹ est le *t*-butyldiméthylsilyle ; et/ou
- dans lequel le composé de formule (III) est dissous dans du toluène ou un éther cyclique ; et/ou
- comprenant également la protection d'un composé de formule (V) pour obtenir les composés de formule (IV) 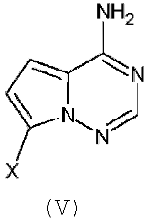 dans lequel
X est hydrogène, brome ou iode
dans un solvant approprié et en présence d'une base appropriée par l'ajout d'un agent protecteur choisi dans le groupe constitué par le chlorure de *t*-butyldiméthylsilyle, le chlorure de triisopropylsilyle et le chlorure de *t*-butyldiphénylsilyle, de préférence
- dans l'étape de protection, le solvant approprié pour un composé de formule (V) est un éther cyclique, et la base appropriée est un alkyllithium, un hydroxyde alcalin ou un alcoolate alcalin, et/ou
- l'étape de protection comprend également une recristallisation dans un alcane supérieur, et/ou
- dans le composé de formule (V), X est hydrogène.

9. Procédé selon l'une quelconque des revendications 1 à 8, dans lequel la réaction de couplage d'un composé de formule (IV) et d'un composé de formule (III) est réalisée en présence d'un composé de formule (VI) dans lequel n est égal à 2, 3 ou 4.

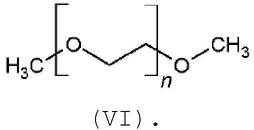

10. Procédé selon la revendication 9, dans lequel le composé de formule (VI) est dissous dans un éther cyclique, et/ou dans lequel, dans les composés de formule (VI), n est 2 ou 3.

11. Procédé selon la revendication 10, dans lequel, dans les composés de formule (VI), n est 2.

12. Procédé selon l'une quelconque des revendications 1 à 8, dans lequel la réaction de couplage est réalisée selon un procédé à flux continu dans lequel un système de deux microréacteurs à flux continu séquentiels (1 et 2) est utilisé comprenant :
- un microréacteur 1 comprenant un micromélangeur et un réacteur à serpentin pour mélanger le composé de formule (IV) avec le réactif de lithiation et les faire réagir pendant un temps de séjour de 20 à 45 s pour obtenir un intermédiaire lithié correspondant,
- un microréacteur 2 comprenant un micromélangeur et un réacteur à serpentin pour mélanger ledit intermédiaire lithié avec un composé de formule (III) et les faire réagir pendant un temps de séjour de 70 à 150 s pour obtenir le composé de formule (II),
- des stockages d'entrée, des pompes et des modules d'injection fournissant trois entrées distinctes A, B et C qui sont connectées aux deux microréacteurs séquentiels,
- des boucles de pré-refroidissement pour chaque entrée, et
- un bain thermostaté,
dans lequel une solution de l'injecteur A et des solutions de l'injecteur B (microréacteur 1) et de l'injecteur C (microréacteur 2) sont injectées séquentiellement dans un flux de THF sec pendant des périodes de temps prédéterminées, suivies de la collecte du mélange et d'un traitement aqueux de celui-ci, et dans lequel
la solution de l'injecteur A est une solution d'un composé de formule (IV),
la solution de l'injecteur B est une solution d'un réactif de lithiation, et
la solution de l'injecteur C est une solution d'un composé de formule (III).

13. Procédé selon la revendication 12, dans lequel les microréacteurs 1 et 2 et les boucles de pré-refroidissement pour chaque entrée sont thermostatés à -30 °C, et/ou dans lequel la température de réaction dans les microréacteurs est de -10 à -30 °C.

14. Composé de formule (II) 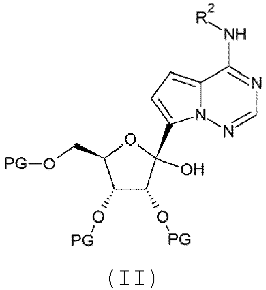 dans lequel
R² est un groupe protecteur choisi dans le groupe constitué par le *t*-butyldiméthylsilyle, le triisopropylsilyle et le *t-*butyldiphénylsilyle, et
PG est un groupe protecteur choisi dans le groupe constitué par le triméthylsilyle, le *t*-butyldiméthylsilyle, le *t-*butyldiphénylsilyle, le méthylméthoxy, le tétrahydropyrane, le *t*-butyle, l'allyle, le benzyle, l'acétyle, le pivaloyle et le benzoyle, ou deux groupes protecteurs sur des atomes de carbone adjacents peuvent être combinés pour former un groupe -C(R³)₂-dans lequel R³ est H, ou un groupe alkylène en C₁₋₆.

15. Composé de formule (IV) 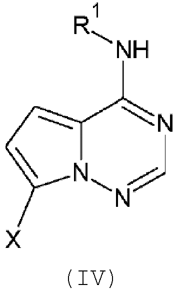 dans lequel
X est hydrogène, brome ou iode, et
R¹ est un groupe protecteur choisi dans le groupe constitué par le *t*-butyldiméthylsilyle, le triisopropylsilyle et le *t-*butyldiphénylsilyle.
